# EUROPEAN PATENT APPLICATION

(11) **EP 1 413 314 A1**
(43) Date of publication of application: **28.04.2004**
(21) Application number: 02743739.1
(22) Date of filing: 27.06.2002
(51) Int. Cl.: A61K 45/00, A61K 48/00, A61P 25/00, A61P 25/16, A61P 25/28, A61P 43/00

(54) **MEDICINAL COMPOSITIONS**

(30) Priority: 27.06.2001 JP 2001195471
(71) Applicant: Japan Science and Technology Agency, Kawaguchi-shi, Saitama 332-0012 (JP); TAISHO PHARMACEUTICAL CO., LTD, Tokyo 170-8633 (JP)
(72) Inventor: TOHYAMA, Masaya, Osaka 565-0081 (JP); KATAYAMA, Taiichi, Osaka 563-0024 (JP); MANABE, Takayuki, Osaka 567-0072 (JP); IMAIZUMI, Kazunori, Nara 630-0101 (JP); IKEDA, Yoko, Taisho Pharmaceutical Co., Ltd., Tokyo 170-8633 (JP)
(74) Representative: VOSSIUS & PARTNER
(86) International application number: PCT/JP2002/006461
(87) International publication number: WO 2003/002146

(57) **Abstract**

To provide a means useful for treating or preventing a disease such as a brain disorder or a neurodegenerative disease even more efficiently and even more sustainedly. The present invention relates to an inhibitor capable of inhibiting a binding between HMG-I protein and exon 5 of presenilin-2 mRNA, an agent for suppressing neuronal death, capable of suppressing neuronal death, a pharmaceutical composition which is useful for treatment or prevention of a disease caused by the generation of a splice variant that lacks exon 5 of presenilin-2 mRNA, a method for treating or preventing the disease and a use of the inhibitor.

SEQUENCE LISTING

<110> Japan Science and Technology Corporation

<110> TAISHO PHARMACEUTICAL CO., LTD.

<120> Pharmaceutical composition

<130> 02-032-PCT

<140>
<141>

<150> JP 2001-195471
<151> 2001-06-27

<160> 68

<170> PatentIn Ver. 2.1

<210> 1
<211> 10
<212> RNA
<213> Artificial Sequence

<220>

<223> Description of Artificial Sequence: a sequence of binding region

<400> 1
gcucuacaag                                                                10

<210> 2
<211> 11
<212> RNA
<213> Artificial Sequence

<220>
<223> Description of Artificial Sequence: a sequence of binding region

<400> 2
gcugcuacaa g                                                              11

<210> 3
<211> 42
<212> RNA
<213> Artificial Sequence

<220>
<223> Description of Artificial Sequence: a sequence of RNA probe No.5

<400> 3
ugguggugcu cuacaaguac cgcugcuaca aggugaggcc cu                            42

<210> 4
<211> 21
<212> RNA
<213> Artificial Sequence

<220>
<223> Description of Artificial Sequence: a sequence of RNA probe No.7

<400> 4
gcucuacaag gcugcuacaa g                                                    21

<210> 5
<211> 19
<212> RNA
<213> Artificial Sequence

<220>
<223> Description of Artificial Sequence: a sequence of RNA probe No.9

<400> 5
ugguggugcu cuacaagua                                                       19

<210> 6
<211> 25
<212> RNA

<213> Artificial Sequence

<220>
<223> Description of Artificial Sequence: a sequence of RNA probe No.10

<400> 6
gcucuacaag uaccgcugcu acaag                                        25

<210> 7
<211> 23
<212> RNA
<213> Artificial Sequence

<220>
<223> Description of Artificial Sequence: a sequence of RNA probe No.11

<400> 7
ccgcugcuac aaggugaggc ccu                                          23

<210> 8
<211> 25
<212> RNA
<213> Artificial Sequence

<220>
<223> Description of Artificial Sequence: a sequence of RNA probe No.8

<400> 8

gcucuaaaag uaccgcugcu aaaag 25

<210> 9

<211> 20

<212> DNA

<213> Artificial Sequence

<220>

<223> Description of Artificial Sequence: asequence for primer ps251

<400> 9

attcagacct ctctgcggcc 20

<210> 10

<211> 20

<212> DNA

<213> Artificial Sequence

<220>

<223> Description of Artificial Sequence: a sequence of primer ps231

<400> 10

aagcgggagc caaagtctgg 20

<210> 11

<211> 20

<212> DNA

<213> Artificial Sequence

<220>

<223> Description of Artificial Sequence: a seqeunce for primer ps252

<400> 11

gttcgtggtg cttccagagg                                                    20

<210> 12

<211> 20

<212> DNA

<213> Artificial Sequence

<220>

<223> Description of Artificial Sequence: a sequence of primer ps233

<400> 12

ggaccactct gggaggtaca                                                    20

<210> 13

<211> 60

<212> DNA

<213> Artificial Sequence

<220>

<223> Description of Artificial Sequence: a sequence of DNA probe No.1

<400> 13

tctggatcct gccttctccc tcagcatcta cacgacattc actgaggaca cgaattcaga 60

<210> 14

<211> 60

<212> DNA

<213> Artificial Sequence

<220>

<223> Description of Artificial Sequence: a sequence of DNA probe No.2

<400> 14

tctggatcct gaggacacac cctcggtggg ccagcgcctc ctcaactccg tgaattcaga 60

<210> 15

<211> 60

<212> DNA

<213> Artificial Sequence

<220>

<223> Description of Artificial Sequence: a sequence of DNA probe No.3

<400> 15

tctggatccc aactccgtgc tgaacaccct catcatgatc agcgtcatcg tgaattcaga 60

<210> 16

<211> 60

<212> DNA

<213> Artificial Sequence

<220>

<223> Description of Artificial Sequence: a sequence of DNA probe No.4

<400> 16

ctcggatcct gatcagcgtc atcgtggtta tgaccatctt cttggtggtg cgaattcgag 60

<210> 17

<211> 60

<212> DNA

<213> Artificial Sequence

<220>

<223> Description of Artificial Sequence: a sequence of DNA probe No.5

<400> 17

tctggatcct ggtggtgctc tacaagtacc gctgctacaa ggtgaggccc tgaattcaga 60

<210> 18

<211> 24

<212> DNA

<213> Artificial Sequence

<220>

<223> Description of Artificial Sequence: a sequence of DNA probe No.6

<400> 18

gatccgacca tcttcttggt ggtg 24

<210> 19

<211> 27

<212> DNA

<213> Artificial Sequence

<220>

<223> Description of Artificial Sequence: a sequence of DNA probe No.7

<400> 19

gatccgctct acaaggctgc tacaagg 27

<210> 20

<211> 31

<212> DNA

<213> Artificial Sequence

<220>

<223> Description of Artificial Sequence: a sequence of DNA probe No.8

<400> 20

gatccgctct aaaagtaccg ctgctaaaag g 31

<210> 21

<211> 37

<212> DNA

<213> Artificial Sequence

<220>

<223> Description of Artificial Sequence: a sequence of DNA probe No.9

<400> 21

tctggatcct ggtggtgctc tacaagtaga attcaga 37

<210> 22

<211> 43

<212> DNA

<213> Artificial Sequence

<220>

<223> Description of Artificial Sequence: a sequence of DNA probe No.10

<400> 22
tctggatccg ctctacaagt accgctgcta caaggaattc aga                43

<210> 23
<211> 41
<212> DNA
<213> Artificial Sequence

<220>
<223> Description of Artificial Sequence: a sequence of DNA probe No.11

<400> 23
tctggatccc cgctgctaca aggtgaggcc ctgaattcag a                41

<210> 24
<211> 49
<212> RNA
<213> Artificial Sequence

<220>
<223> Description of Artificial Sequence: a sequence of 5'-amino-2'-O-me
thyl oligo RNA

<400> 24
aucuucuugg uggugcucua caaguaccgc ugcuacaagg ugaggcccu                49

<210> 25

<211> 18

<212> RNA

<213> Artificial Sequence

<220>

<223> Description of Artificial Sequence: a sequence of RNA probe No.6

<400> 25

gaccaucuuc uugguggu 18

<210> 26

<211> 72

<212> RNA

<213> Artificial Sequence

<220>

<223> Description of Artificial Sequence: a sequence of RNA probe No.12

<400> 26

ugguggugcu cuacaaguac cgcugcuaca aggugaggcc cuggcccugc ccuccagcca 60

cgcuucucuc cg 72

<210> 27

<211> 25

<212> RNA

<213> Artificial Sequence

<220>
<223> Description of Artificial Sequence: a sequence of 2'-O-methyl olig
o RNA corresponding to a sequence of HMG-I protein binding region which
is present in SEQ ID NO: 24

<400> 27
gcucuacaag uaccgcugcu acaag                                          25

<210> 28
<211> 26
<212> DNA
<213> Artificial Sequence

<220>
<223> Description of Artificial Sequence: a sequence of synthesized DNA
containing HMG-I recognition motif

<400> 28
ggttcattct gcggtatggt tatctg                                         26

<210> 29
<211> 31
<212> DNA
<213> Artificial Sequence

<220>

<223> Description of Artificial Sequence: a sequence of synthesized DNA
containing HMG-I recognition motif

<400> 29
atctgtaatt ccatggtggc tactggaact a                                    31

<210> 30
<211> 31
<212> DNA
<213> Artificial Sequence

<220>
<223> Description of Artificial Sequence: a sequence of synthesized DNA
containing HMG-I recognition motif

<400> 30
gtggaggctg ccgcgagtta tcacagtata g                                    31

<210> 31
<211> 30
<212> DNA
<213> Artificial Sequence

<220>

<223> Description of Artificial Sequence: a sequence of synthesized DNA
containing HMG-I recognition motif

<400> 31
acctaggtta ttctgcggta tggttatctg                                        30

<210> 32
<211> 30
<212> DNA
<213> Artificial Sequence

<220>
<223> Description of Artificial Sequence: a sequence of synthesized DNA
containing HMG-I recognition motif

<400> 32
gggtgcctag ctccggaaca tggttattga                                        30

<210> 33
<211> 31
<212> DNA
<213> Artificial Sequence

<220>
<223> Description of Artificial Sequence: a sequence of synthesized DNA
containing HMG-I recognition motif

<400> 33
cgttctcttt cggaaacggt ttttgagcac g                                    31

<210> 34
<211> 30
<212> DNA
<213> Artificial Sequence

<220>
<223> Description of Artificial Sequence: a sequence of synthesized DNA
containing HMG-I recognition motif

<400> 34
tgaggcatga aggtaattac tgcatattga                                      30

<210> 35
<211> 30
<212> DNA
<213> Artificial Sequence

<220>
<223> Description of Artificial Sequence: a sequence of synthesized DNA
containing HMG-I recognition motif

<400> 35

ggccgcgtgt agatattggg atttggtgtg 30

<210> 36
<211> 28
<212> DNA
<213> Artificial Sequence

<220>
<223> Description of Artificial Sequence: a sequence of synthesized DNA
containing HMG-I recognition motif

<400> 36
cgttctcttt cggaaatttt tgagcacg 28

<210> 37
<211> 31
<212> DNA
<213> Artificial Sequence

<220>
<223> Description of Artificial Sequence: a sequence of synthesized DNA
containing HMG-I recognition motif

<400> 37
tgaccaagta aatccgtccc tcactagtga a 31

<210> 38
<211> 31
<212> DNA
<213> Artificial Sequence

<220>
<223> Description of Artificial Sequence: a sequence of synthesized DNA
containing HMG-I recognition motif

<400> 38
gccatgtttt tagataattg tgcttaggcg t                                    31

<210> 39
<211> 31
<212> DNA
<213> Artificial Sequence

<220>
<223> Description of Artificial Sequence: a sequence of synthesized DNA
containing HMG-I recognition motif

<400> 39
tctgaggcat gaaggtaatt actgcatcat t                                    31

<210> 40
<211> 30

<212> DNA
<213> Artificial Sequence

<220>
<223> Description of Artificial Sequence: a sequence of synthesized DNA
containing HMG-I recognition motif

<400> 40
tatccagtgg ataattcccc aatgttatct                                        30

<210> 41
<211> 31
<212> DNA
<213> Artificial Sequence

<220>
<223> Description of Artificial Sequence: a sequence of synthesized DNA
containing HMG-I recognition motif

<400> 41
cgttccgacc aatgggttcg gataatacgt t                                      31

<210> 42
<211> 31
<212> DNA
<213> Artificial Sequence

<220>
<223> Description of Artificial Sequence: a sequence of synthesized DNA
containing HMG-I recognition motif

<400> 42
cttctgcggt ataaggtggg ttggtgtgct c                                    31

<210> 43
<211> 31
<212> DNA
<213> Artificial Sequence

<220>
<223> Description of Artificial Sequence: a sequence of synthesized DNA
containing HMG-I recognition motif

<400> 43
gaggtgtcag gcgacaccta aaaattcacg c                                    31

<210> 44
<211> 32
<212> DNA
<213> Artificial Sequence

<220>

<223> Description of Artificial Sequence: a sequence of synthesized DNA
containing HMG-I recognition motif

<400> 44
atatcctgcc aaaaatttgt tctcggggct ga                                32

<210> 45
<211> 31
<212> DNA
<213> Artificial Sequence

<220>
<223> Description of Artificial Sequence: a sequence of synthesized DNA
containing HMG-I recognition motif

<400> 45
tgtttcgcgt aaagtattac gttgctcttt t                                 31

<210> 46
<211> 31
<212> DNA
<213> Artificial Sequence

<220>
<223> Description of Artificial Sequence: a sequence of synthesized DNA
containing HMG-I recognition motif

<400> 46
gtcgggcctg tattttggtt gctattttga a                                    31

<210> 47
<211> 31
<212> DNA
<213> Artificial Sequence

<220>
<223> Description of Artificial Sequence: a sequence of synthesized DNA
containing HMG-I recognition motif

<400> 47
gcgagtcggg cctgtatttt ggttgctatt t                                    31

<210> 48
<211> 32
<212> DNA
<213> Artificial Sequence

<220>
<223> Description of Artificial Sequence: a sequence of synthesized DNA
containing HMG-I recognition motif

<400> 48

ttgaattagg gtgctatatt tttgcatttt ga 32

<210> 49
<211> 31
<212> DNA
<213> Artificial Sequence

<220>
<223> Description of Artificial Sequence: a sequence of synthesized DNA
containing HMG-I recognition motif

<400> 49
gtcgggcctg tattttggtt gctattttga a 31

<210> 50
<211> 31
<212> DNA
<213> Artificial Sequence

<220>
<223> Description of Artificial Sequence: a sequence of synthesized DNA
containing HMG-I recognition motif

<400> 50
gcgagtcggg cctgtatttt ggttgctatt t 31

<210> 51
<211> 32
<212> DNA
<213> Artificial Sequence

<220>
<223> Description of Artificial Sequence: a sequence of synthesized DNA
containing HMG-I recognition motif

<400> 51
ccactttgaa ttagggtgct atatttttgc at                                32

<210> 52
<211> 31
<212> DNA
<213> Artificial Sequence

<220>
<223> Description of Artificial Sequence: a sequence of synthesized DNA
containing HMG-I recognition motif

<400> 52
gtggataaat tccccaatgt tatcttatga a                                 31

<210> 53
<211> 32

<212> DNA
<213> Artificial Sequence

<220>
<223> Description of Artificial Sequence: a sequence of synthesized DNA
containing HMG-I recognition motif

<400> 53
ctttgaatta gggtgctata tttttgcatt tt                                32

<210> 54
<211> 35
<212> DNA
<213> Artificial Sequence

<220>
<223> Description of Artificial Sequence: a sequence of synthesized DNA
containing HMG-I recognition motif

<400> 54
cgtgtttcgc cagaaccgca ctagagcaat ttgaa                             35

<210> 55
<211> 33
<212> DNA
<213> Artificial Sequence

<220>

<223> Description of Artificial Sequence: a sequence of synthesized DNA
containing HMG-I recognition motif

<400> 55
ctttgaatta gggtgctata tggtttgcat ttt                                    33

<210> 56
<211> 31
<212> DNA
<213> Artificial Sequence

<220>
<223> Description of Artificial Sequence: a sequence of synthesized DNA
containing HMG-I recognition motif

<400> 56
gtggataaat tccccaatgt tatcttatga a                                      31

<210> 57
<211> 31
<212> DNA
<213> Artificial Sequence

<220>

<223> Description of Artificial Sequence: a sequence of synthesized DNA
containing HMG-I recognition motif

<400> 57
ggtgccgata cgtaactcac cgcattcttg g                                    31

<210> 58
<211> 31
<212> DNA
<213> Artificial Sequence

<220>
<223> Description of Artificial Sequence: a sequence of synthesized DNA
containing HMG-I recognition motif

<400> 58
cgttccgacc aatgggttcg gataatacgt t                                    31

<210> 59
<211> 31
<212> DNA
<213> Artificial Sequence

<220>
<223> Description of Artificial Sequence: a sequence of synthesized DNA
containing HMG-I recognition motif

<400> 59
ggtgccgata cgtaactcac cgcattttgg c 31

<210> 60
<211> 31
<212> DNA
<213> Artificial Sequence

<220>
<223> Description of Artificial Sequence: a sequence of synthesized DNA
containing HMG-I recognition motif

<400> 60
ggtgccgata cgtaatcacc gcattcttgg c 31

<210> 61
<211> 31
<212> DNA
<213> Artificial Sequence

<220>
<223> Description of Artificial Sequence: a sequence of synthesized DNA
containing HMG-I recognition motif

<400> 61

cactttgaat tagggtgcta tatgttttgc a 31

<210> 62
<211> 31
<212> DNA
<213> Artificial Sequence

<220>
<223> Description of Artificial Sequence: a sequence of synthesized DNA
containing HMG-I recognition motif

<400> 62
ttgaattagg gtgctatatg ttttgcattt t 31

<210> 63
<211> 31
<212> DNA
<213> Artificial Sequence

<220>
<223> Description of Artificial Sequence: a sequence of synthesized DNA
containing HMG-I recognition motif

<400> 63
cgttctcttt cggaaacggt ttttgagcac g 31

<210> 64

<211> 31

<212> DNA

<213> Artificial Sequence

<220>

<223> Description of Artificial Sequence: a sequence of synthesized DNA

containing HMG-I recognition motif

<400> 64

aggttcgtgc acccggacca cagttttttc a 31

<210> 65

<211> 70

<212> DNA

<213> Artificial Sequence

<220>

<223> Description of Artificial Sequence: a sequence of synthesized DNA

used in in vitro DNA selection assay

<220>

<223> n is g or a or t or c

<400> 65

ggtgatcaga ttctgatcca nnnnnnnnnn nnnnnnnnnn nnnnnnnnnn ntgaagcttg 60

gatccgtcgc                                                    70

<210> 66
<211> 14
<212> DNA
<213> Artificial Sequence

<220>
<223> Description of Artificial Sequence: a sequence of synthesized DNA
 containing HMG-I recognition motif

<400> 66
gcgrgwwatt tcgc                                               14

<210> 67
<211> 39
<212> DNA
<213> Artificial Sequence

<220>
<223> Description of Artificial Sequence: a sequence of primer for the u
se for in vitro DNA selection assay

<400> 67
gtaatacgac tcactatagg gtgatcagat tctgatcca                    39

<210> 68

<211> 19

<212> DNA

<213> Artificial Sequence

<220>

<223> Description of Artificial Sequence: a sequence of primer for the u
se for in vitro DNA selection assay

<400> 68

gcgacggatc caagcttca                                                    19

## Description

### TECHNICAL FIELD

The present invention relates to an inhibitor capable of inhibiting a binding between HMG-I protein and exon 5 of presenilin-2 mRNA, an agent for suppressing neuronal death, capable of suppressing neuronal death, a pharmaceutical composition which is useful for treatment or prevention of a disease caused by the generation of a splice variant that lacks exon 5 of presenilin-2 mRNA, a method for treating or preventing the disease and a use of the inhibitor.

### BACKGROUND ART

In Alzheimer's disease (AD), which is one of neurodegenerative diseases, and the diseases of over 90% of the AD patients are classified as sporadic Alzheimer's disease (sAD). It has been known that an aberrant protein encoded by a variant of presenilin-2 gene (PS2V) is present in apoptotic pyramidal cells of each of cerebral cortex and hippocampal CA1 region of the sAD patient [Sato, N. et al., *J. Biol. Chem*., **276**, 2108-2114 (2001)].

It is shown that PS2V is induced by hypoxic stimulation in cultured human neuroblastoma SK-N-SH cells, and is dependent on a new protein synthesis in each of oxidative stress and SK-N-SH cells [Sato, N. et al., *J. Neurochem*., **72**, 2498-2505 (1999)].

It has been known that a glucose-regulated protein (GRP78), which is endoplasmic reticulum (ER) stress-responsive molecular chaperone, is reduced by the above PS2V, and the generation of β-amyloid protein is increased in PS2V-expressed cells [Sato, N. et al., *J. Biol. Chem*., **276**, 2108-2114 (2001)].

However, although the possibility that PS2V is one of important factors for neuronal death has been suggested, the details of the mechanisms of the generation of PS2V and incidence of the disease have not been sufficiently known in the present situation. Also, a method for treatment which is effective for the disease caused by the generation of the above PS2V is desired in the present situation.

### DISCLOSURE OF INVENTION

An object of the present invention is to provide an inhibitor capable of inhibiting a binding between HMG-I protein and exon 5 of presenilin-2 mRNA. Another object of the present invention is to provide an agent for suppressing neuronal death, capable of inhibiting a binding between HMG-I protein and exon 5 of presenilin-2 mRNA and suppressing aberrant splicing that lacks exon 5 of presenilin-2, thereby suppressing neuronal death. A further object of the present invention is to provide a pharmaceutical composition capable of achieving at least one of being capable of suppressing cell death, particularly neuronal death, being excellent in introduction efficiency into a cell, being excellent in stability in a living body, and treating or preventing a disease caused by the generation of a splice variant that lacks exon 5 of presenilin-2 mRNA, particularly a brain disorder, a neurodegenerative disease or the like. A still another object of the present invention is to provide a method of treating or preventing a disease caused by the generation of splice variant that lacks exon 5 of presenilin-2 mRNA, by which the treatment or prevention of a disease caused by the generation of splice variant that lacks exon 5 of presenilin-2 mRNA, particularly a brain disorder or a neurodegenerative disease, can be carried out even more efficiently and even more sustainedly.

Concretely, the present invention relates to:
[1] an inhibitor for a binding between HMG-I protein and presenilin-2 gene, comprising a compound capable of inhibiting a binding between HMG-I protein and exon 5 of presenilin-2 mRNA as an active ingredient;
[2] the inhibitor according to the above [1], wherein the compound is one kind selected from the group consisting of:
   (A) an RNA molecule comprising the nucleotide sequence shown in SEQ ID NO: 1 and/or the nucleotide sequence shown in SEQ ID NO: 2, wherein the RNA molecule is capable of binding to the HMG-I protein;
   (B) a DNA molecule comprising a DNA sequence corresponding to the nucleotide sequence shown in SEQ ID NO: 1 and/or a DNA sequence corresponding to the nucleotide sequence shown in SEQ ID NO: 2, wherein the DNA molecule is capable of binding to the HMG-I protein;
   (C) an antisense molecule complementary to either the RNA molecule of the above (A) or the DNA molecule of the above (B);
   (D) an analog molecule which is an analog of either the RNA molecule of the above (A) or the DNA molecule of the above (B), wherein the analog molecule is capable of binding to the HMG-I protein; and
   (E) an analog molecule of the antisense molecule of the above (C);
[3] the inhibitor according to the above [2], wherein the compound is one kind selected from the group consisting of:
   (a) an RNA molecule comprising the nucleotide sequence shown in any one of SEQ ID NO: 3, 4, 5, 6 or 7, wherein the RNA molecule is capable of binding to the HMG-I protein;
   (b) a DNA molecule comprising a DNA sequence corresponding to the nucleotide sequence shown in any one of SEQ ID NO: 3, 4, 5, 6 or 7, wherein the DNA molecule is capable of binding to the HMG-I protein;
   (c) an antisense molecule complementary to either the RNA molecule of the above (a) or the DNA molecule of the above (b);
   (d) an analog molecule which is an analog of either the RNA molecule of the above (a) or the DNA molecule of the above (b), wherein the analog molecule is capable of binding to the HMG-I protein; and
   (e) an analog molecule of the antisense molecule of the above (c);
[4] the inhibitor according to the above [1] or [2], wherein the compound is 2'-O-methyl oligo RNA molecule having the nucleotide sequence shown in SEQ ID NO: 27;
[5] an agent for suppressing neuronal death, comprising as an active ingredient: an inhibitor for a binding between HMG-I protein and presenilin-2 gene, wherein the inhibitor comprises a compound capable of inhibiting a binding between HMG-I protein and exon 5 of presenilin-2 mRNA as an active ingredient;
[6] the agent for suppressing neuronal death according to the above [5], wherein the compound is one kind selected from the group consisting of:
   (A) an RNA molecule comprising the nucleotide sequence shown in SEQ ID NO: 1 and/or the nucleotide sequence shown in SEQ ID NO: 2, wherein the RNA molecule is capable of binding to the HMG-I protein;
   (B) a DNA molecule comprising a DNA sequence corresponding to the nucleotide sequence shown in SEQ ID NO: 1 and/or a DNA sequence corresponding to the nucleotide sequence shown in SEQ ID NO: 2, wherein the DNA molecule is capable of binding to the HMG-I protein;
   (C) an antisense molecule complementary to either the RNA molecule of the above (A) or the DNA molecule of the above (B);
   (D) an analog molecule which is an analog of either the RNA molecule of the above (A) or the DNA molecule of the above (B), wherein the analog molecule is capable of binding to the HMG-I protein; and
   (E) an analog molecule of the antisense molecule of the above (C);
[7] the agent for suppressing neuronal death according to the above [6], wherein the compound is one kind selected from the group consisting of:
   (a) an RNA molecule comprising the nucleotide sequence shown in any one of SEQ ID NO: 3, 4, 5, 6 or 7, wherein the RNA molecule is capable of binding to the HMG-I protein;
   (b) a DNA molecule comprising a DNA sequence corresponding to the nucleotide sequence shown in any one of SEQ ID NO: 3, 4, 5, 6 or 7, wherein the DNA molecule is capable of binding to the HMG-I protein;
   (c) an antisense molecule complementary to either the RNA molecule of the above (a) or the DNA molecule of the above (b);
   (d) an analog molecule which is an analog of either the RNA molecule of the above (a) or the DNA molecule of the above (b), wherein the analog molecule is capable of binding to the HMG-I protein; and
   (e) an analog molecule of the antisense molecule of the above (c);
[8] the agent for suppressing neuronal death according to the above [5] or [6], wherein the compound is 2'-O-methyl oligo RNA molecule having the nucleotide sequence shown in SEQ ID NO: 27;
[9] a pharmaceutical composition comprising as an active ingredient:
   an inhibitor for a binding between HMG-I protein and presenilin-2 gene, comprising a compound capable of inhibiting a binding between HMG-I protein and exon 5 of presenilin-2 mRNA as an active ingredient; or
   an agent for suppressing neuronal death, comprising the inhibitor as an active ingredient;
[10] the pharmaceutical composition according to the above [9], wherein the compound is one kind selected from the group consisting of:
   (A) an RNA molecule comprising the nucleotide sequence shown in SEQ ID NO: 1 and/or the nucleotide sequence shown in SEQ ID NO: 2, wherein the RNA molecule is capable of binding to the HMG-I protein;
   (B) a DNA molecule comprising a DNA sequence corresponding to the nucleotide sequence shown in SEQ ID NO: 1 and/or a DNA sequence corresponding to the nucleotide sequence shown in SEQ ID NO: 2, wherein the DNA molecule is capable of binding to the HMG-I protein;
   (C) an antisense molecule complementary to either the RNA molecule of the above (A) or the DNA molecule of the above (B);
   (D) an analog molecule which is an analog of either the RNA molecule of the above (A) or the DNA molecule of the above (B), wherein the analog molecule is capable of binding to the HMG-I protein; and
   (E) an analog molecule of the antisense molecule of the above (C);
[11] the pharmaceutical composition according to the above [10], wherein the compound is one kind selected from the group consisting of:
   (a) an RNA molecule comprising the nucleotide sequence shown in any one of SEQ ID NO: 3, 4, 5, 6 or 7, wherein the RNA molecule is capable of binding to the HMG-I protein;
   (b) a DNA molecule comprising a DNA sequence corresponding to the nucleotide sequence shown in any one of SEQ ID NO: 3, 4, 5, 6 or 7, wherein the DNA molecule is capable of binding to the HMG-I protein;
   (c) an antisense molecule complementary to either the RNA molecule of the above (a) or the DNA molecule of the above (b);
   (d) an analog molecule which is an analog of either the RNA molecule of the above (a) or the DNA molecule of the above (b), wherein the analog molecule is capable of binding to the HMG-I protein; and
   (e) an analog molecule of the antisense molecule of the above (c);
[12] the pharmaceutical composition according to the above [9] or [10], wherein the compound is 2'-O-methyl oligo RNA molecule having the nucleotide sequence shown in SEQ ID NO: 27;
[13] the pharmaceutical composition according to any one of the above [9] to [12], wherein the pharmaceutical composition possesses an action of suppressing aberrant splicing;
[14] the pharmaceutical composition according to any one of the above [9] to [13], wherein the pharmaceutical composition possesses an action of suppressing generation of a splicing variant that lacks exon 5 of presenilin-2 mRNA;
[15] the pharmaceutical composition according to any one of the above [9] to [14], wherein the pharmaceutical composition is usable for treating or preventing a brain disorder or a neurodegenerative disease caused by generation of a splicing variant that lacks exon 5 of presenilin-2 mRNA;
[16] the pharmaceutical composition according to the above [15], wherein the brain disorder is an ischemic brain disorder or a brain neurodegenerative disease;
[17] the pharmaceutical composition according to the above [16], wherein the brain disorder is a disease selected from the group consisting of cerebrovascular dementia, Parkinson's syndrome and Alzheimer disease;
[18] the pharmaceutical composition according to the above [15], wherein the neurodegenerative disease is amyotrophic lateral sclerosis;
[19] use of the inhibitor of any one of the above [1] to 4 or the agent for suppressing neuronal death of any one of the above [5] to [8], for manufacture of a pharmaceutical composition for administering to an individual with a disease caused by generation of a splicing variant that lacks exon 5 of presenilin-2 mRNA, thereby treating or preventing the disease caused by generation of a splicing variant that lacks exon 5 of presenilin-2 mRNA; and
[20] a method for treating or preventing a disease caused by generation of a splicing variant that lacks exon 5 of presenilin-2 mRNA, characterized by administering the inhibitor of any one of the above [1] to [4] or the agent for suppressing neuronal death of any one of the above [5] to [8] in a therapeutically effective amount to an individual with a disease caused by generation of a splicing variant that lacks exon 5 of presenilin-2 mRNA.

### BRIEF DESCRIPTION OF THE DRAWINGS

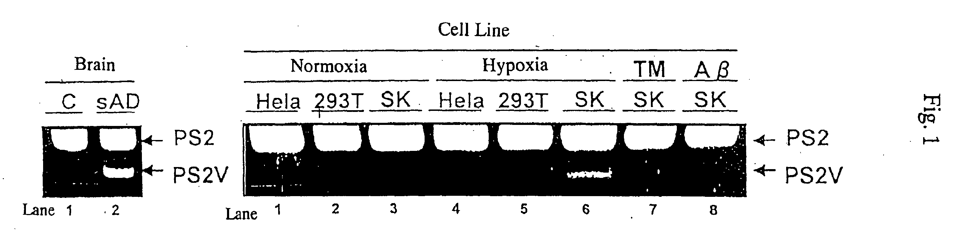

Fig. 1 is a diagram showing expression of PS2V in a sAD brain and cultured cell lines. The left panel is the results of separating an amplified product from a representative sAD brain or from an age-matching control brain on a polyacrylamide gel and visualizing by staining with ethidium bromide. The right panel is the results of subjecting total RNAs to RT-PCR, wherein the total RNAs derived from various cells under normoxia or various stresses such as hypoxic exposure, tunicamycin (TM) and β-amyloid, separating the resulting PCR product on a polyacrylamide gel, and visualizing by staining with ethidium bromide. In the figure, the solid arrows indicate points of the molecular weights corresponding to PS2 (upper part) and PS2V (lower part), respectively. These experiments are repeated at least four times using different cell cultures, and the same results are obtained.

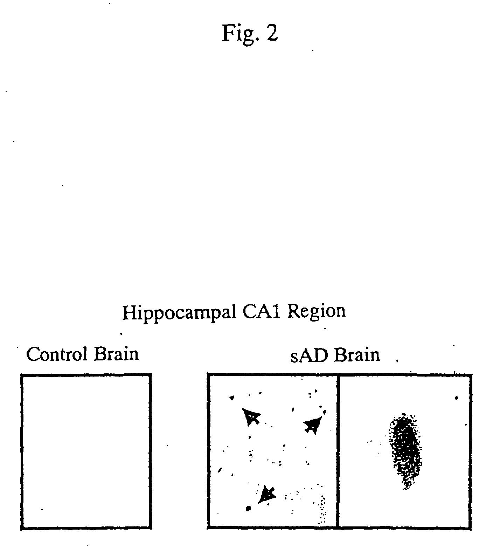

Fig. 2 is a diagram showing the results of immunohistochemical detection of PS2V in each of a sAD brain and a control brain. Brain sections for control and sAD of 10 µm in thickness were prepared and thereafter subjected to immunohistochemical detection of PS2V using an anti-SSMAG antibody. These results were obtained by analyzing at least three different kinds of control and AD brains, and the same results were obtained.

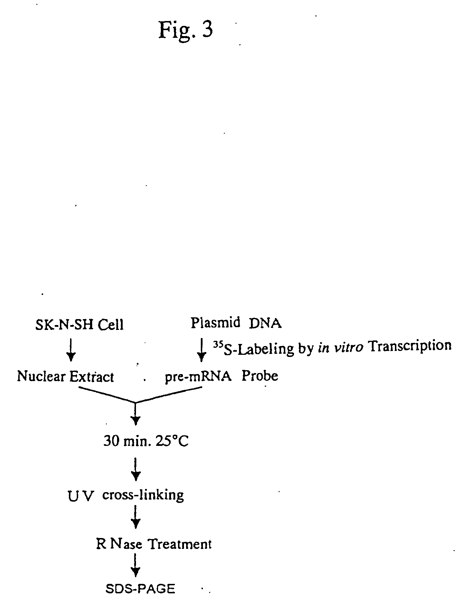

Fig. 3 is a diagram showing a scheme of pre-mRNA binding assay. SK-N-SH cells are collected 21 hours after normoxia or hypoxic stimulation, followed by a preparation of a nuclear extract, and subsequent binding reaction with each probe of PS2 pre-mRNA fragments. The reaction solution is irradiated with ultraviolet (UV) rays. Thereafter, the probe is digested with RNase A, and the digest is subjected to SDS treatment. The SDS-treated sample is separated by SDS-PAGE assay, and thereafter a binding-positive band is detected by using a Bas imaging plate.

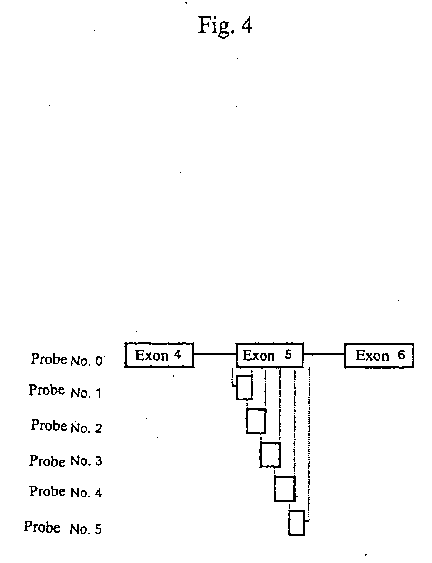

Fig. 4 is a schematic diagram of PS2 pre-mRNA probes. In the figure, six kinds of probes of PS2 pre-mRNA fragment for the binding assay are schematically shown. The region circumscribed with a rectangular frame and the underlined portion each represents exon and intron. All the probes are labeled by *in vitro* transcription.

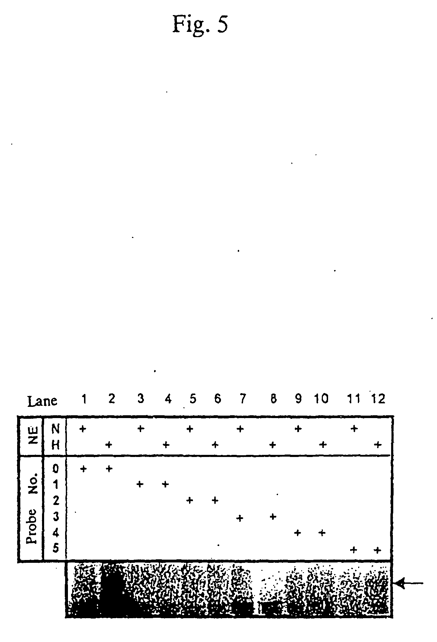

Fig. 5 is a diagram showing the results of a pre-mRNA binding experimental method. SK-N-SH cells were exposed to normoxia or hypoxia, and then collected after 21 hours of stimulation. A nuclear extract from the collected cells was analyzed by the pre-mRNA binding assay using each radioisotope-labeled probe. Next, the nuclear extract was subjected to SDS-PAGE on a polyacrylamide gel on (10 to 20%) concentration gradient and thereafter exposed on an imaging plate for Bas. These experiments were repeated at least four times using different cell cultures, and the same results were obtained.

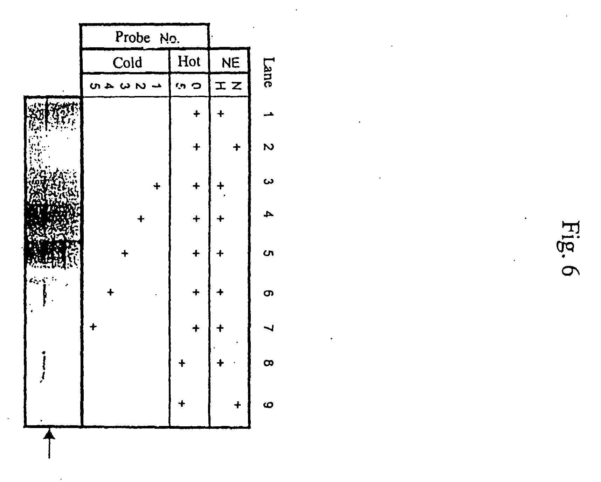

Fig. 6 is a diagram showing the results of the pre-mRNA binding assay. SK-N-SH cells were exposed to normoxia or hypoxia and then collected after 21 hours of stimulation. The nuclear extract was pre-incubated under binding conditions together with each ³⁵S-unlabeled cold probe. The pre-mRNA binding assay using the pre-incubated reaction solution was initiated by adding each ³⁵S-labeled hot probe. Next, the mixture was subjected to SDS-PAGE on 15% polyacrylamide gel, followed by exposure to a Bas imaging plate. These experiments were repeated at least four times using different cell cultures, and the same results were obtained.

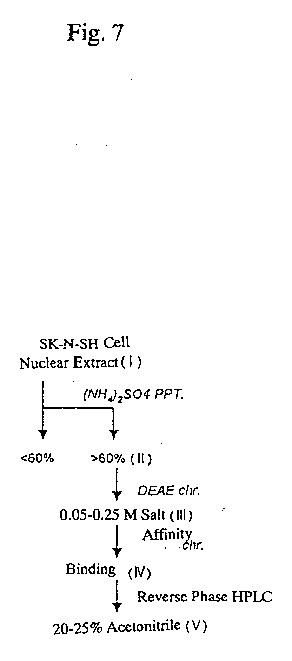

Fig. 7 shows a purification profile of a fraction having a binding activity to Probe No. 5 which is derived from a nuclear extract of human neuroblastoma SK-N-SH cells under hypoxic stress. The purification method is shown in italics. Individual fractions are shown in Roman numerals.

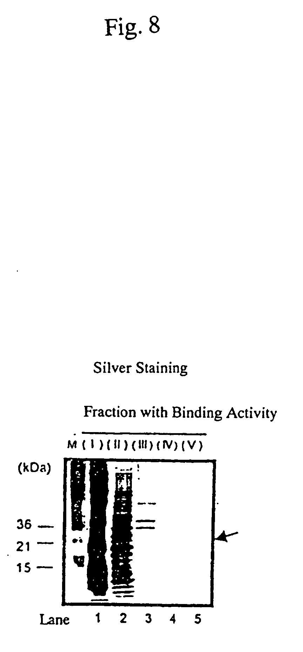

Fig. 8 is a diagram showing the results of analysis of proteins (25 µl/fraction) in each purification stage shown in Fig. 7 by 15% SDS-PAGE and silver staining. In the figure, M is a molecular weight (kDa) marker.

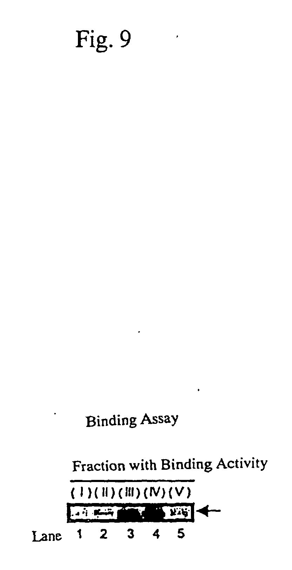

Fig. 9 is a diagram showing the binding activity of ³⁵S-labeled Probe No. 5 to a fraction (15 µl/fraction) in each purification stage.

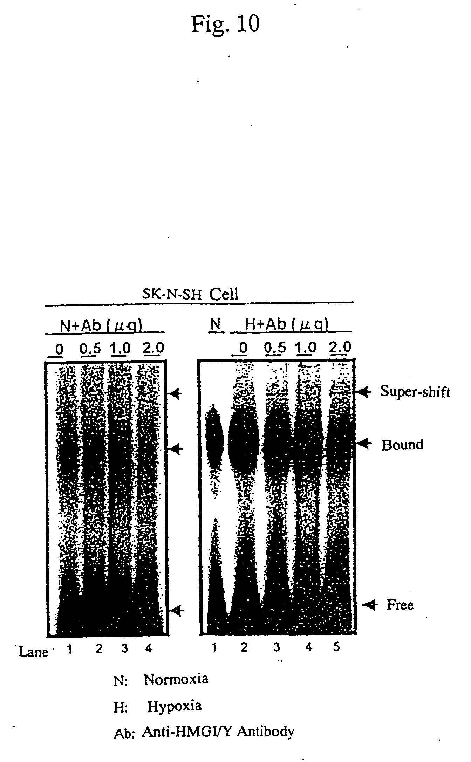

Fig. 10 is a diagram showing the results of super shift assay using an antibody against HMG-I/Y protein. SK-N-SH cells were collected after 21 hours of normoxia (left) or hypoxic (right) stimulation. Prior to the reaction with the radioisotope-labeled Probe No. 5, the nuclear extract was incubated in a usual buffer in the presence or absence of the antibody against the HMG-I/Y protein. Next, the sample was subjected to gel retardation electrophoresis and autoradiography. These experiments were repeated at least four times using different cell cultures, and the same results were obtained.

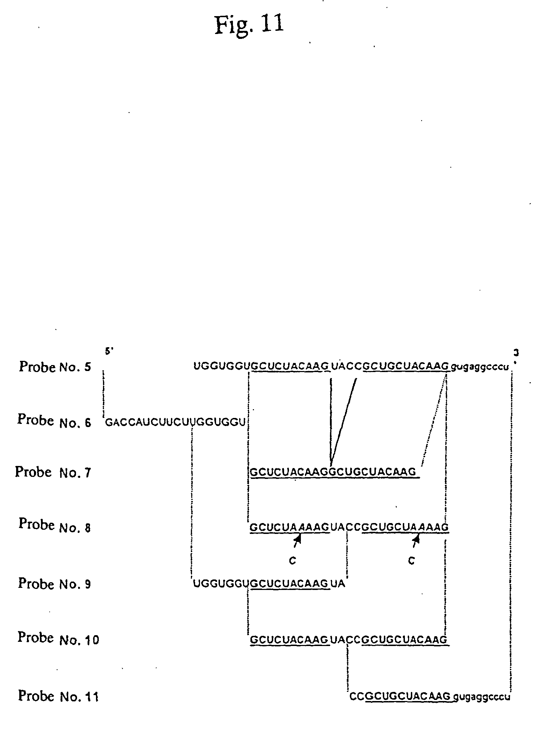

Fig. 11 is a diagram showing seven kinds of structures of PS2 pre-mRNA. In the figure, exon is shown in capital letters, and intron in small letters. Two analogous sequences are shown in bold letters with underlines.

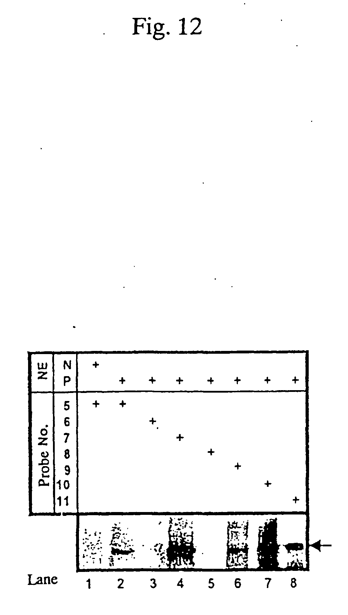

Fig. 12 is a diagram showing the results of examination of a binding site of HMG-I protein in PS2 pre-mRNA. SK-N-SH cells are exposed to normoxia or hypoxia and then collected after 21 hours of stimulation. By the pre-mRNA binding assay using each radioisotope-labeled probe, the nuclear extract from the collected cells is analyzed. Next, the nuclear extract is subjected to SDS-PAGE, followed by exposure to a Bas imaging plate. In the figure, the solid arrow indicates a point of the corresponding molecular weight. These experiments are repeated at least four times using different cell culture media, and the same results are obtained.

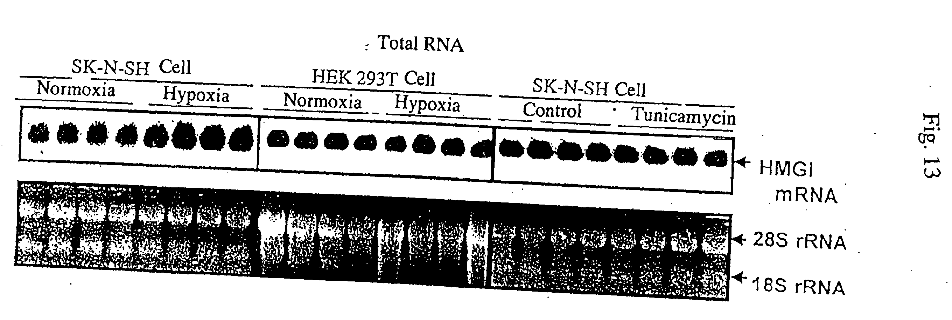

Fig. 13 is a diagram showing the results of examination of the effects of various stresses on expression of HMG-I mRNA (HMGI mRNA in the figure) and the protein corresponding to the mRNA in the cultured cells by Northern blot analysis. The left panel and the central panel each shows the analytical results of the cell lines collected after 21 hours of exposure to normoxia or hypoxia. The right panel shows the analytical results of SK-N-SH cells collected 24 hours after treatment with tunicamycin. The total RNA is electrophoresed on a formaldehyde-formamide gel and subjected to Northern blot assay using a radioisotope-labeled HMG-I cDNA probe. In the figure, a point of the molecular weight is indicated by a solid arrow.

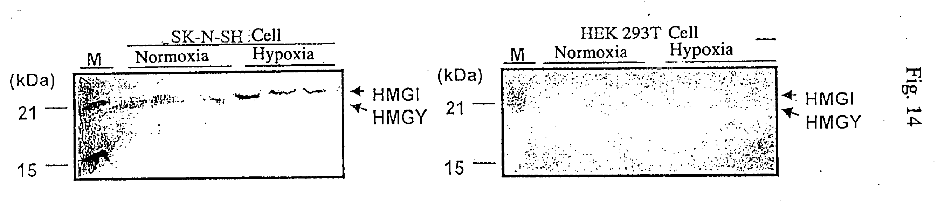

Fig. 14 is a diagram showing the results of examination of the effects of various stresses on expression of HMG-I mRNA and the protein corresponding to the mRNA in the cultured cells by Western blot analysis. Each cell line is exposed to normoxia or hypoxia, and thereafter collected after 21 hours of stimulation. The resulting nuclear extract is subjected to SDS-PAGE and an immunoblot assay using an antibody against HMG-I/Y protein. In the figure, the solid arrows indicate points of the molecular weights corresponding to the HMG-I (upper panel, "HMGI" in the figure) and HMG-Y (lower panel, "HMGY" in the profile) proteins.

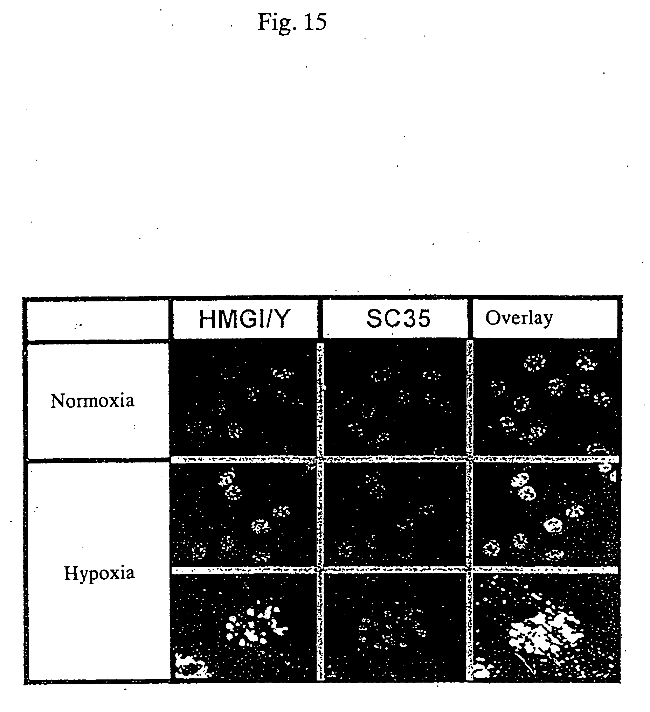

Fig. 15 is a diagram showing the effects of hypoxia on cellular localization of immunoreactive HMG-I protein in SK-N-SH cells. SK-N-SH cells are exposed to normoxia or hypoxia for 21 hours. The cells are immunostained with an anti-HMG-I/Y ("HMGI/Y" in the figure) antibody and anti-SC35 antibody for primary immunoreaction and stained with an FITC-bound secondary antibody and a Cy3-conjugated secondary antibody. These experiments are repeated at least four times using different cell cultures, and the same results are obtained.

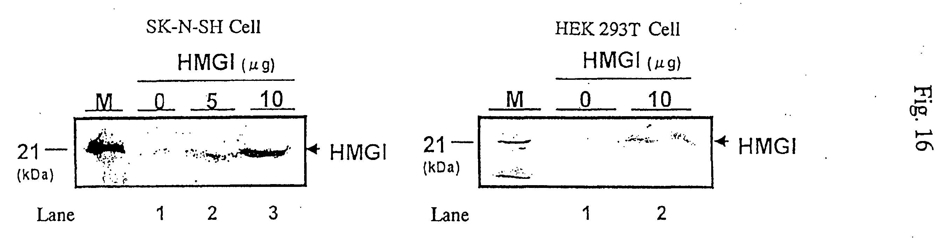

Fig. 16 is a diagram showing the results of examination of the effect of transient expression of HMG-I or HMG-Y on exon 5 skipping in PS2 gene in the cultured cells. Each cell line was transfected with mock or HMG-I, followed by collection 24 hours after the transfection. The nuclear extract was subjected to SDS-PAGE and immunoblot assay using an antibody against HMG-I/Y. The solid arrow indicates a point of the molecular weight corresponding to HMG-I protein ("HMGI" in the figure). These experiments were repeated at least four times using different cell culture media, and the same results were obtained.

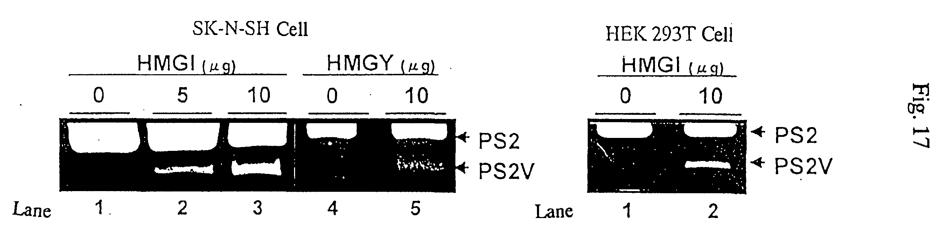

Fig. 17 is a diagram showing the results of examination of the effect of the transient expression of HMG-I or HMG-Y on exon 5 skipping of PS2 gene in the cultured cells. Each cell line was transfected with different amounts of mock, HMG-I ("HMGI" in the figure) or HMG-Y ("HMGY" in the figure), and collected 24 hours after the transfection. The total RNA from the collected cells was subjected to RT-PCR according to a method of a literature [Sato et al., *J. Neurochem*., **72**, 2498-2505 (1999)]. The amplified product was separated on a polyacrylamide gel and visualized by staining with ethidium bromide. The solid arrows indicate points of the molecular weights corresponding to PS2 (upper band) and PS2V (lower part). These experiments were repeated at least 4 times using different cell culture media, and the same results were obtained.

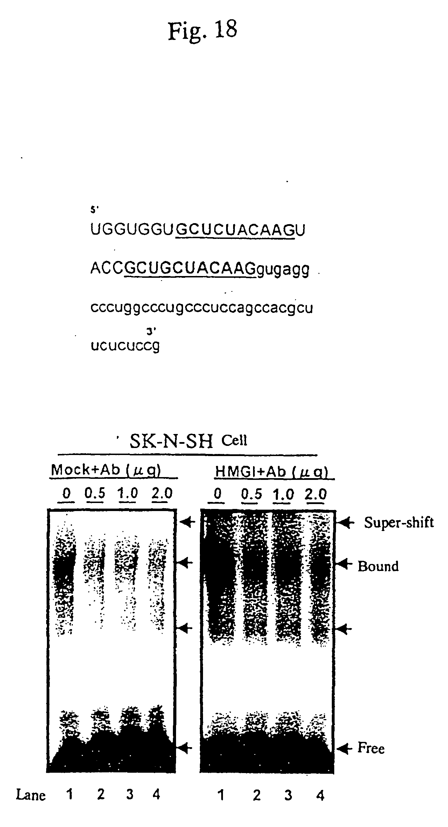

Fig. 18 is a diagram showing the results of examination of the effect of HMG-I ("HMGI" in the figure) on the binding of U1 (70K) snRNP to PS2 pre-mRNA. Prior to the nuclear extract derived from the collected cells was reacted with (³²P-labeled) Probe No. 12 shown in the upper panel, the nuclear extract was incubated in a usual buffer in the presence or absence of an antibody against HMG-I/Y protein. Next, the sample was subjected to gel retardation electrophoresis and autoradiography. In the figure, the solid arrows indicate the free probe, bound probe and super shift probe. Also, in the figure, Ab represents an anti-U1 snRNP antibody. These experiments were repeated at least four times using different cell cultures, and the same results were obtained.

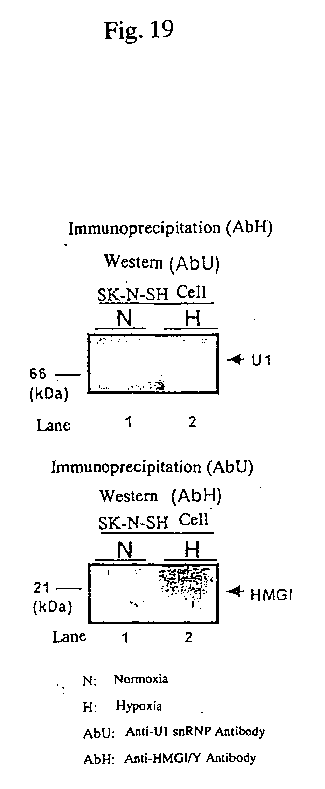

Fig. 19 is a diagram showing an interaction between HMG-I/Y and U1 (70 K) snRNP. The upper panel shows the results of immunoblot assay with an anti-U1 (70 K) snRNP antibody. SK-N-SH cells exposed to normoxia or hypoxia were collected after 24 hours of stimulation to prepare a nuclear extract. The nuclear extract was immunoprecipitated with an anti-HMG-I/Y antibody ("anti-HMGI/Y antibody" in the figure), followed by immunoblot assay with an anti-U1 (70 K) snRNP antibody. The lower panel shows the results of immunoblot assay with an anti-HMG-I/Y antibody. The nuclear extract was immunoprecipitated using an anti-U1 (70 K) snRNP antibody and then subjected to immunoblot assay using an anti-HMG-I/Y antibody. These experiments were repeated at least three times using different cell cultures, and the same results are obtained.

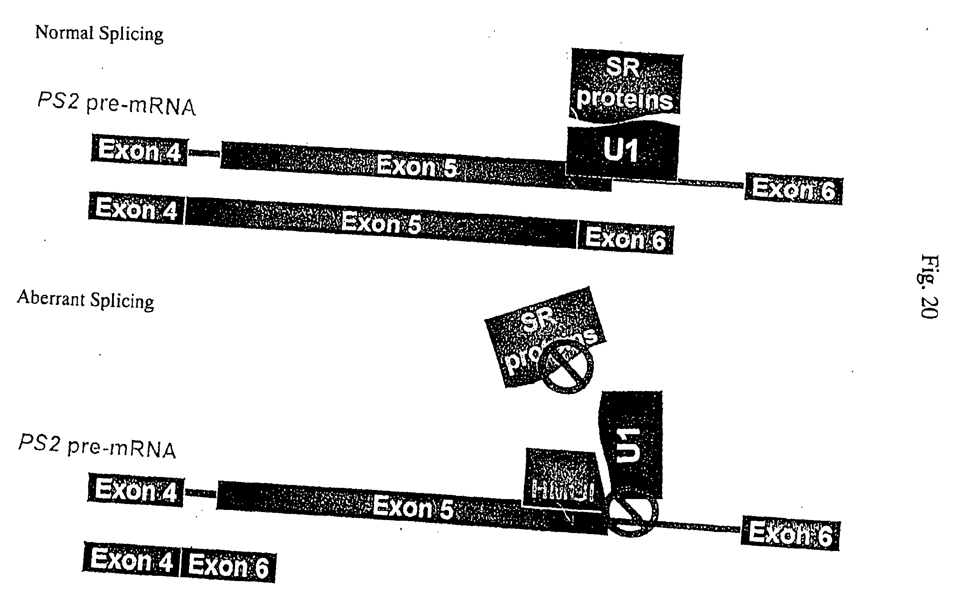

Fig. 20 is a schematic diagram of a hypothesis for a mechanism of aberrant splicing on PS2 pre-mRNA.

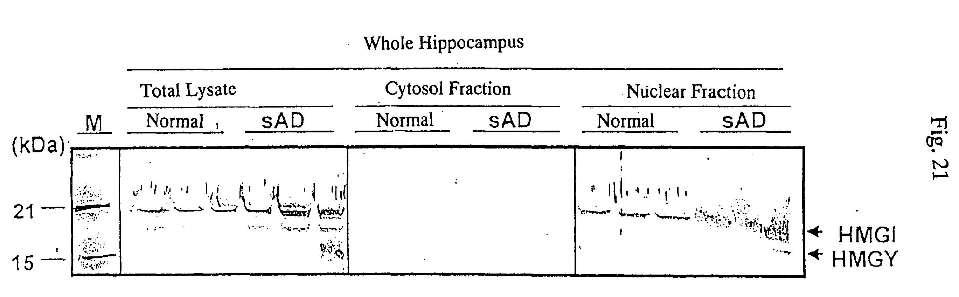

Fig. 21 is a diagram showing the results of examination of expression of HMG-I/Y protein in the sAD brain by Western blot analysis. The total lysate, cytosol fraction and nuclear fraction were prepared from three different sAD brains and age-matching control hippocampuses. These fractions were subjected to SDS-PAGE and to immunoblot assay using an antibody against HMG-I/Y protein. The solid arrows indicate points of the molecular weights corresponding to HMG-I (upper band, "HMGI" in the figure) and HMG-Y (lower part, "HMGY" in the figure) proteins.

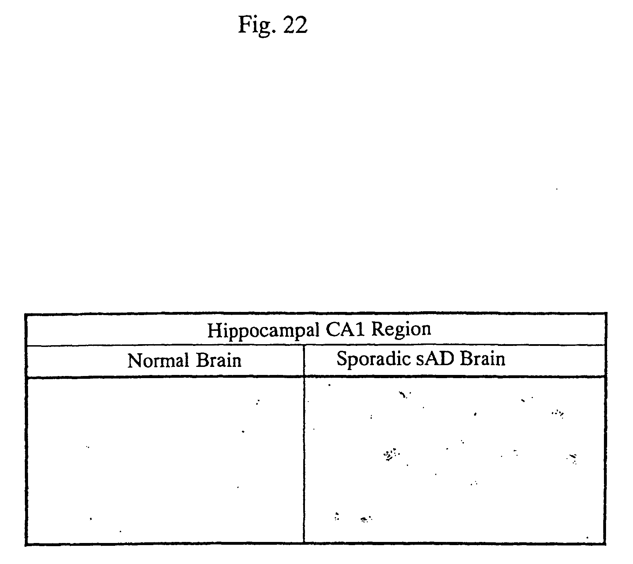

Fig. 22 is a diagram showing the results of examination of expression of HMG-I/Y protein in the sAD brain by immunohistochemistry. At least three kinds of different control and AD brains were analyzed, and the same results were always obtained. Brain sections for control and sAD of 10 µm in thickness were prepared, and subjected to an immunohistochemical detection of immunoreactive HMG-I/Y. At least three different kinds of control and AD brains were analyzed, and the same results were always obtained.

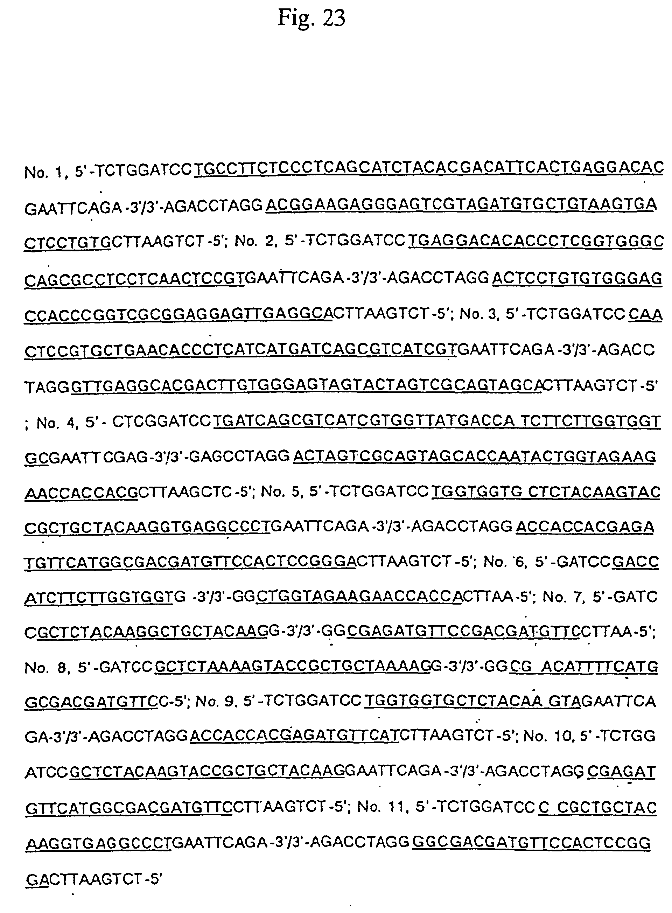

Fig. 23 is a diagram showing each synthesized oligonucleotide, which was carried out by a conventional method. Annealing was carried out using two complementary oligonucleotides having bases in length of 24-mer or 60-mer to give each of double-stranded DNA No. 1 to No. 11. In the figure, each fragment of exon 5 of PS2 is underlined and shown in bold letters in each oligonucleotide.

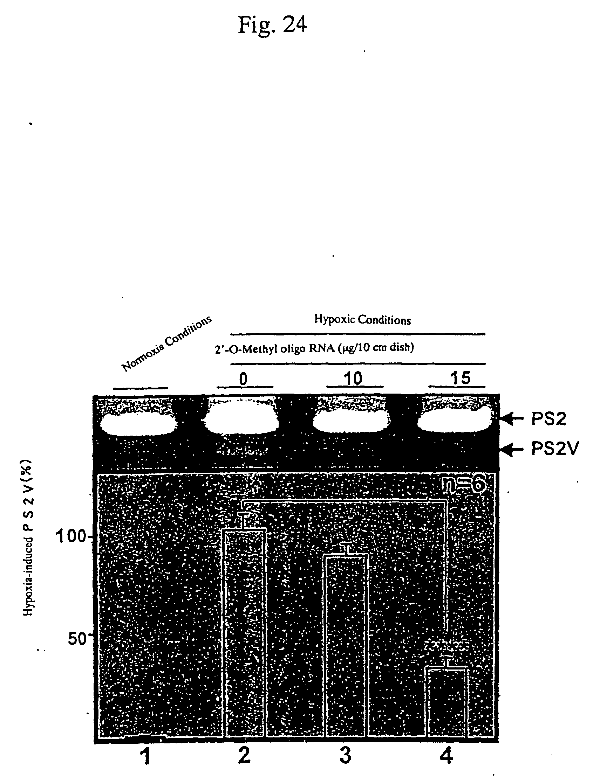

Fig. 24 is a diagram showing the results of examination of the effect of the suppression of generation of a splicing variant that lacks exon 5 of presenilin-2 gene by 2'-O-methyl oligo RNA.

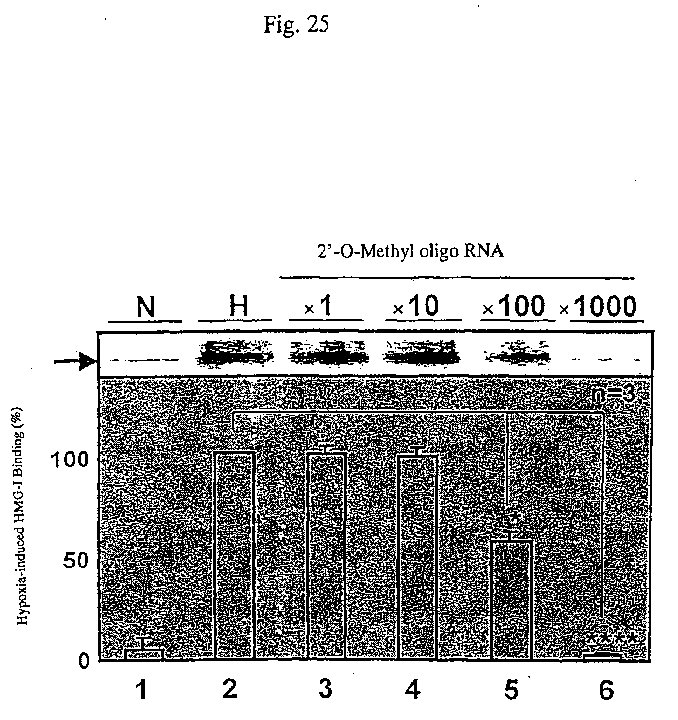

Fig. 25 is a diagram showing the results of examination of effect of 2'-O-methyl oligo RNA in a binding to hypoxia-induced HMG-I *in vitro*. In the figure, "N" in lane 1 represents normoxia conditions, and "H" in lane 2 represents hypoxic conditions. Also, the amount of 2'-O-methyl oligo RNA used is 0.5 µg for lane 3, 5 µg for lane 4, 50 µg for lane 5 and 500 µg for lane 6.

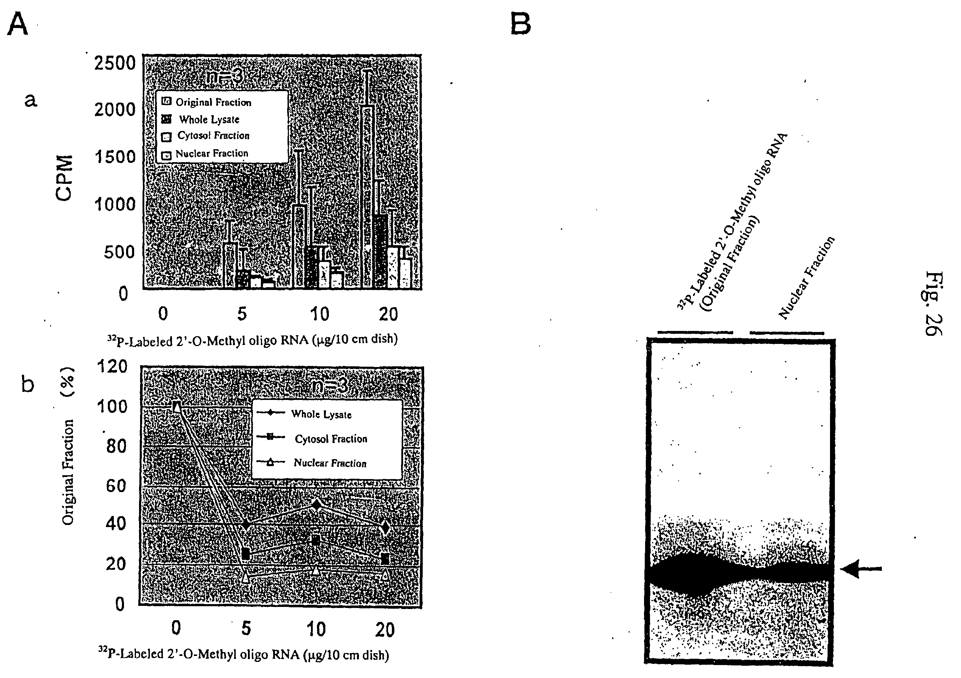

Fig. 26 is a diagram showing the results of examination of uptake, stability and introduction efficiency of 2'-O-methyl oligo RNA in a cell. In the panel A, panel a shows an amount of uptake of 2'-O-methyl oligo RNA in each fraction of SK-N-SH cell, and panel b shows introduction efficiency of the above 2'-O-methyl oligo RNA into SK-N-SH cell. The panel B shows the results of examination of stability of 2'-O-methyl oligo RNA at a point of 48 hours after the introduction of the above 2'-O-methyl oligo RNA into SK-N-SH cell.

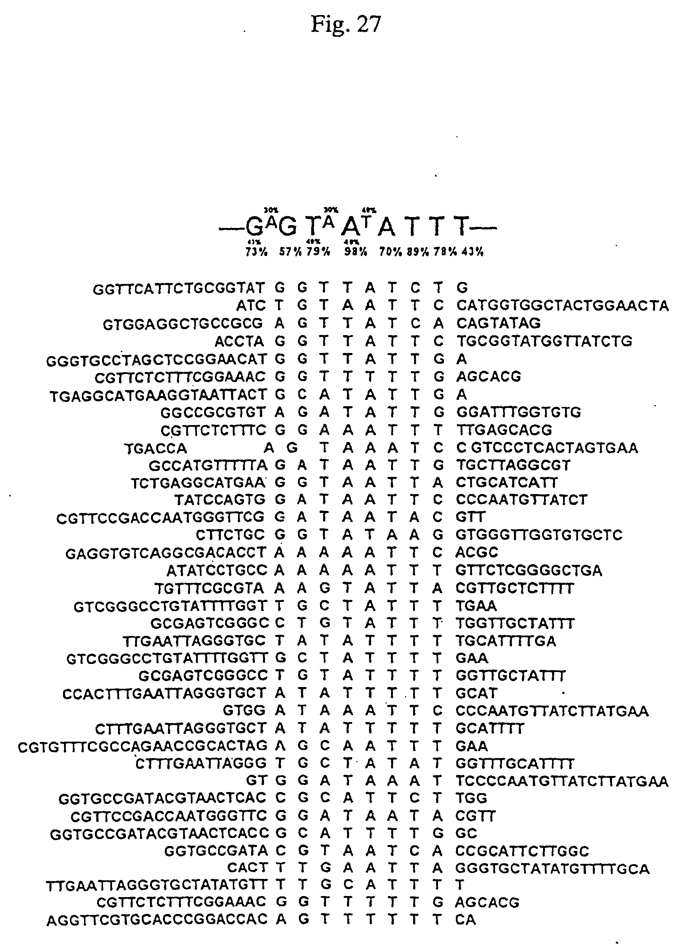

Fig. 27 is a diagram showing sequences of synthetic DNAs for determining a DNA recognition motif of HMG-I. The uppermost row shows a consensus sequence of the HMG-I recognition motif.

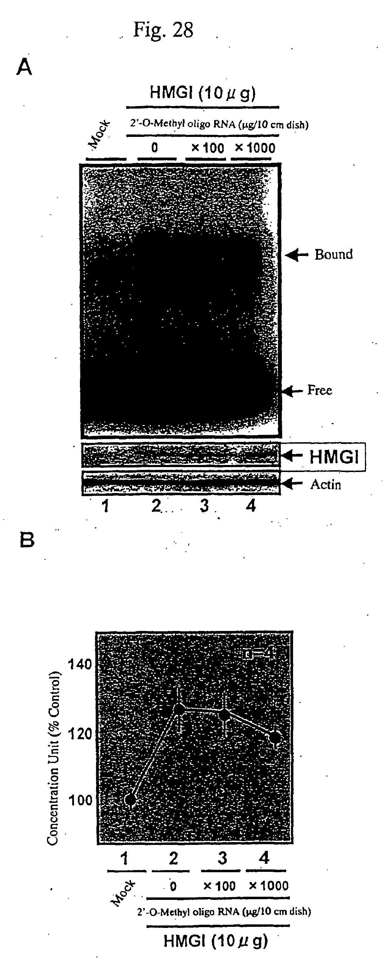

Fig. 28 is a diagram showing the results of examination of the action of 2'-O-methyl oligo RNA on the binding of HMG-I to DNA in the nuclear extract derived from SK-N-SH cells. The panel A shows the results of gel shift assay, and the panel B shows data numerically expressing those of the panel A.

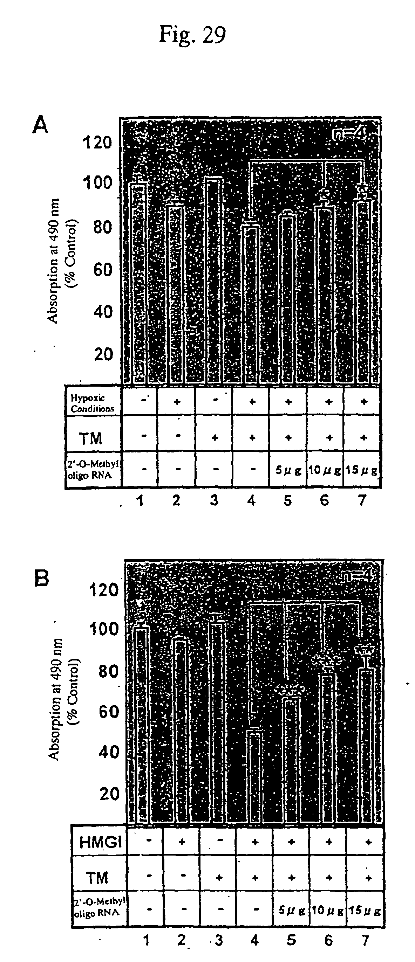

Fig. 29 shows the results of examination of the action of 2'-O-methyl oligo RNA against cell death. The panel A shows the results of examination on cell death induced with hypoxic conditions or tunicamycin, and the panel B shows the results of examination on cell death induced with transient expression of HMG-I and tunicamycin.

### BEST MODE FOR CARRYING OUT THE INVENTION

One of the features of the inhibitor of the present invention resides in that the inhibitor comprises a compound capable of inhibiting a binding between HMG-I protein and exon 5 of presenilin-2 mRNA as an active ingredient. Since the inhibitor of the present invention comprises the above compound, there is exhibited an excellent effect that the inhibitor can inhibit the binding between the HMG-I protein and presenilin-2 mRNA. Also, according to the inhibitor of the present invention, there is exhibited an excellent effect that an aberrant splicing of the above presenilin-2 mRNA, particularly an aberrant splicing that lacks exon 5 of presenilin-2 can be suppressed. Further, since the inhibitor of the present invention can suppress an aberrant splicing that lacks exon 5 of presenilin-2, there is further exhibited an excellent effect that neuronal death can be suppressed.

The presenilin-2 gene is a gene which is thought to be one of causative genes in Alzheimer's disease. A protein encoded by the above presenilin-2 gene is a membrane protein existing in the endoplasmic reticulum Golgi body, the protein relating to amyloid metabolism or deposition. The above presenilin-2 gene is constituted by 12 exons, out of which 10 exons encode a protein [see, for instance, Levy-Lahad et al., *Genomics*, **34**, 198-204 (1996); Levy-Lahad et al., *Science,* **269**, 973-977 (1995); Rogaev et al., *Nature*, **376**, 775-778 (1995), and the like].

The "presenilin-2 gene" as used herein is intended to mean that any of mRNA and DNA (for instance, genomic DNA, cDNA), each encoding presenilin-2 protein are included. A nucleotide sequence of the presenilin-2 gene and the amino acid sequence thereof are described in, for instance, Accession Nos. XM002127, XM002128, XP002127 in GenBank or the like.

The "compound capable of inhibiting a binding between HMG-I protein and exon 5 of presenilin-2 mRNA" used in the present invention may be, for instance, either a compound that specifically antagonizes a binding at a particular site, thereby completely inhibiting the binding, or a compound that specifically antagonizes a binding at a particular site, thereby reducing a binding strength of the binding.

Concretely, the above "particular site" includes, for instance, ① a region comprising the nucleotide sequence shown in SEQ ID NO: 1; ② a region comprising the nucleotide sequence shown in SEQ ID NO: 2; ③ a region comprising the nucleotide sequence shown in SEQ ID NO: 1 and the nucleotide sequence shown in SEQ ID NO: 2; ④ a region comprising continuous sequences on DNA, corresponding to each of the above ① to ③; and the like.

Also, in the present invention, the above "particular site" also includes (i) a region comprising a continuous sequence involved in a binding between the HMG-I protein and the presenilin-2 gene in an amino acid sequence of the HMG-I protein; (ii) a spatial region formed by a plural amino acid residues existing in the HMG-I protein, wherein the amino acid residues are involved in a binding between the HMG-I protein and the presenilin-2 gene.

The above "amino acid residues ... involved in a binding" includes, for instance, residues by which an ability of binding to the presenilin-2 gene of the resulting protein is disappeared when substituted with other amino acid residues; and the like.

The nucleotide sequence shown in SEQ ID NO: 1 and the nucleotide sequence shown in SEQ ID NO: 2 mentioned above, are sequences existing in mRNA of the presenilin-2 gene, wherein the nucleotide sequences found by the present inventors that are important in a binding to the HMG-I protein. The present invention is based on the above findings of the present inventors.

In the present invention, the above SEQ ID NO: 1 or 2 may be a sequence having a mutation of at least one residue in the above SEQ ID NO: 1 or 2, wherein the sequence exhibits an ability of binding to the HMG-I protein, and a DNA sequence corresponding thereto, or may be a sequence having a sequence identity of at least 90%, preferably 95% or more to the nucleotide sequence shown in SEQ ID NO: 1 or the nucleotide sequence shown in SEQ ID NO: 2, wherein the sequence exhibits an ability of binding to the HMG-I protein.

The above compound may be a low-molecular compound or a macromolecular compound. The above compound includes, for instance, nucleic acids such as RNA and DNA; organic synthetic compounds and the like. The above nucleic acid may be single-stranded or double-stranded, and may be linear or cyclic.

The above compound includes, for instance, a compound capable of blocking a region consisting of an amino acid sequence existing in the HMG-I protein, wherein the amino acid sequence is involved in a binding between the HMG-I protein and the presenilin-2 gene (for instance, nucleic acids, nucleic acid analogs and the like); a compound capable of blocking a spatial region formed by a plural amino acid residues existing in the HMG-I protein, wherein the amino acid residues are involved in a binding between the HMG-I protein and the presenilin-2 gene (for instance, an organic synthetic compound fitting to a spatial region, or the like); a compound capable of blocking a region comprising a nucleotide sequence existing in exon 5 of presenilin-2 mRNA, wherein the region is a region in which the HMG-I protein and the presenilin-2 gene are associated (for instance, nucleic acids, nucleic acid analogs and the like); and the like.

In addition, in the present invention, a compound capable of inhibiting a binding between the HMG-I protein and the presenilin-2 gene via the nucleotide sequence shown in SEQ ID NO: 1 and/or the nucleotide sequence shown in SEQ ID NO: 2 mentioned above is also encompassed within the scope of the present invention.

In the present specification, the "sequence identity" refers to identity in corresponding residues between a nucleic acid molecule comprising the nucleotide sequence shown in SEQ ID NO: 1 or 2 and a nucleic acid molecule to be compared. The above "sequence identity" is intended to include a case where a residue is similar to the residues of the nucleotide sequence shown in SEQ ID NO: 1 or 2 involving a modified base such as a 2'-O-methyl derivative, a deamino derivative (for instance, inosine or the like), an adenosine derivative (for instance, isopentenyladenine, threoninocarbonyladenine or the like), or a sulfur-containing derivative (4-thiouridine, 2-thiopyrimidine). The above "sequence identity" can be determined by comparing two nucleotide sequences aligned in an optimal state over the region of the nucleotide sequence to be compared. The numerical value (percentage) of sequence identity can be calculated by determining identical bases of a nucleic acid existing in both the sequences, determining the number of matching sites, thereafter dividing the above number of matching sites by a total number of bases in the region of the sequence to be compared, and multiplying the obtained numerical value by 100. The algorism for obtaining an optimal alignment and homology includes, for instance, local homology algorism of Smith et al. [*Add. APL. Math*., **2**, 482 (1981)], homology alignment algorism of Needleman et al. [*J. Mol. Biol*., **48**, 443 (1970)], a homology searching method according to Pearson et al. [*Proc. Natl. Acad. Sci. USA*, **85**, 2444 (1988)], and the like. More concretely, there are included a dynamic programming method, a gap penalty method, Smith-Waterman algorism, Good-Kanehisa algorism, BLAST algorism, FASTA algorism and the like.

Concretely, the above compound includes (A) an RNA molecule comprising the nucleotide sequence shown in SEQ ID NO: 1 and/or the nucleotide sequence shown in SEQ ID NO: 2, wherein the RNA molecule is capable of binding to the HMG-I protein. According to the RNA molecule of the above (A), the region of the above (i) or (ii) can be blocked.

Concretely, the RNA molecule of the above (A) includes, for instance, (a) an RNA molecule comprising the nucleotide sequence shown in any one of SEQ ID NO: 3, 4, 5, 6 or 7, wherein the RNA molecule is capable of binding to the HMG-I protein, and the like.

In addition, a nucleic acid derivative obtainable from the RNA molecule of the above (A) is also encompassed within the scope of the present invention, as long as the nucleic acid derivative has an equivalent binding affinity from the viewpoint of the binding affinity between the HMG-I and one kind selected from the group consisting of a nucleic acid comprising the nucleotide sequence shown in SEQ ID NO: 1, a nucleic acid comprising the nucleotide sequence shown in SEQ ID NO: 2, and a nucleic acid comprising the nucleotide sequence shown in SEQ ID NO: 1 and the nucleotide sequence shown in SEQ ID NO: 2.

In other words, in the present invention, the above SEQ ID NO: 1 or 2 may be a sequence having mutation(s) of at least one residue in the above SEQ ID NO: 1 or 2, wherein the sequence is capable of exhibiting an ability of binding to the HMG-I protein, and a DNA sequence corresponding thereto. Alternatively, the above SEQ ID NO: 1 or 2 may be a sequence having at least 90%, preferably 95% or more sequence identity to the above nucleotide sequence shown in SEQ ID NO: 1 or 2, wherein the sequence is capable of exhibiting an ability of binding to the HMG-I protein, and a DNA sequence corresponding thereto. The nucleic acids each having the above sequences are also encompassed within the scope of the present invention.

In the above binding affinity, the binding to the HMG-I protein can be evaluated by a conventional technique used for analysis of protein-nucleic acid interactions, such as Southwestern analysis, gel shift assay, or resonance plasma interaction analysis.

Concretely, the nucleic acid and nucleic acid derivative, for instance, include:
(B) a DNA molecule comprising a DNA sequence corresponding to the nucleotide sequence shown in SEQ ID NO: 1 and/or a DNA sequence corresponding to the nucleotide sequence shown in SEQ ID NO: 2, wherein the DNA molecule is capable of binding to the HMG-I protein;
(C) an antisense molecule complementary to either the RNA molecule of the above (A) or the DNA molecule of the above (B);
(D) an analog molecule which is an analog of either the RNA molecule of the above (A) or the DNA molecule of the above (B), wherein the analog molecule is capable of binding to the HMG-I protein;
(E) an analog molecule of the antisense molecule of the above (C); and the like.

According to the DNA molecule of the above (B), there is expected to block a region in exon 5 of presenilin-2 mRNA, wherein the region is a region in which the HMG-I protein and the presenilin-2 gene are associated. In the above (B), the "DNA sequence corresponding to the nucleotide sequence shown in SEQ ID NO: 1" or the "DNA sequence corresponding to the nucleotide sequence shown in SEQ ID NO: 2" means a complementary strand to a reverse transcriptional product of the sequence shown in SEQ ID NO: 1 or SEQ ID NO: 2. Concretely, the DNA molecule of the above (B) includes, for instance, (b) a DNA molecule comprising a DNA sequence (wherein u is replaced by t) corresponding to the nucleotide sequence shown in any one of SEQ ID NO: 3, 4, 5, 6, or 7, wherein the DNA molecule is capable of binding to the HMG-I protein.

In the above (C), according to the molecule complementary to the RNA molecule of the above (A) (antisense molecule), there is expected to block a region in exon 5 of presenilin-2 mRNA, wherein the region is a region in which the HMG-I protein and the presenilin-2 gene are associated. On the other hand, according to the antisense molecule complementary to the DNA molecule of the above (B), there is expected to block the region of the above (i) or (ii). The antisense molecule shown in the above (C) includes antisense molecules complementary to either the RNA molecule of the above (a) or the DNA molecule of the above (b).

In the above (D), the analog molecule includes an oligonucleotide modified in the RNA molecule of the above (A) or the DNA molecule of the above (B), in order that the oligonucleotide is less likely to be degraded in a living body, including an oligonucleotide having a thiophosphoric acid diester linkage in which an oxygen atom in a phosphoric acid diester linkage is replaced by a sulfur atom (S-oligo); an oligonucleotide in which a phosphoric acid diester linkage is replaced by an uncharged methyl phosphate group; and the like. The analog molecule may be a nucleic acid which is a so-called peptide nucleic acid. Concretely, the above analog molecule includes 2'-O-methyl oligo RNA having the nucleotide sequence shown in SEQ ID NO: 27, 4-thiouridine-containing RNA having the nucleotide sequence shown in SEQ ID NO: 27, 2-thiopyrimidine-containing RNA having the nucleotide sequence shown in SEQ ID NO: 27, and the like. These analog molecules are especially advantageous from the viewpoint of, for instance, being excellent in stability in a living body.

In the above (D), according to the analog molecule which is an analog of the RNA molecule of the above (A), wherein the analog molecule is capable of binding to the HMG-I protein, there is expected to block the region of the above (i) or (ii). In addition, according to the analog molecule which is an analog of the DNA molecule of the above (B), wherein the analog molecule is capable of binding to the HMG-I protein, there is expected to block a region in exon 5 of presenilin-2 mRNA, wherein the region is a region in which the HMG-I protein and the presenilin-2 gene are associated. Concretely, the analog molecule as shown in the above (D) includes, for instance, an analog molecule of either an analog of the RNA molecule of the above (a) or an analog of the DNA molecule of the above (b), wherein the analog molecule is capable of binding to the HMG-I protein.

In the above (E), the analog molecule includes an oligonucleotide modified in the antisense molecule of the above (C), in order that the oligonucleotide is less likely to be degraded in a living body, including an oligonucleotide having a thiophosphoric acid diester linkage in which an oxygen atom in a phosphoric acid diester linkage is replaced by a sulfur atom (S-oligo), an oligonucleotide in which a phosphoric acid diester linkage is replaced by an uncharged methyl phosphate group, or the like. Also, in the case of the above analog molecule, the analog molecule may be a nucleic acid which is a so-called peptide nucleic acid. Concretely, the analog molecule includes (e) an analog molecule of the antisense molecule of the above (c).

When the nucleic acid or nucleic acid derivative is used as the compound used in the inhibitor of the present invention, the nucleic acid or nucleic acid derivative can be obtained by conventional genetic recombination techniques, nucleic acid synthesis methods and site-directed mutagenesis. For instance, the nucleic acid or nucleic acid derivative may be directly synthesized with, for instance, a DNA synthesizer in accordance with a nucleic acid synthesis method generally used in genetic engineering, or may be obtained by synthesizing a nucleic acid comprising the nucleic acid or nucleic acid derivative (particularly a mutant), or a part thereof, and then amplifying the nucleic acid by PCR or with a cloning vector or the like. Further, in the case of a DNA molecule, the DNA molecule obtained by these methods may be cleaved with a restriction enzyme or the like, thereafter the cleaved fragments are ligated to an appropriate vector or the like with a DNA ligase or the like, and the resulting recombinant vector is introduced into an appropriate host to give a transformant from which a nucleic acid may be prepared. Base moieties, sugar moieties or phosphate moieties of the above nucleic acid may be chemically modified in order to obtain a more stable nucleic acid derivative in a cell.

The above site-directed mutagenesis includes, for instance, gapped duplex method [see, for instance, *Nucleic Acids Research*, **12**, 9441-9456 (1984) or the like], Kunkel method [see, for instance, *Proc. Natl. Acad. Sci. USA*, **82**, 488-492 (1985) or the like], a PCR method using primers for mutagenesis, and the like.

The above nucleic acid synthesis method includes triphosphate method, phosphoramidite method, H-phosphonate method and the like.

The pharmacological evaluation of the inhibitor of the present invention can be carried out, for instance, by detecting a binding between the HMG-I protein and the nucleic acid of the above (A), more concretely, a nucleic acid having the nucleotide sequence shown in SEQ ID NO: 4 in the presence of a substance to be tested (substance to be subjected to pharmacological evaluation), or by determining a binding strength of the binding (for instance, binding affinity or the like).

In other words, I) a case where a binding between the HMG-I protein and the nucleic acid is not detected in the presence of the substance to be tested, or II) a case where a binding strength a of a binding between the HMG-I protein and the nucleic acid in the presence of the substance to be tested is lower than a binding strength b of a binding between the HMG-I protein and the nucleic acid in the absence of the substance to be tested, is used as an index showing that the substance to be tested is an inhibitor for a binding between the HMG-I protein and exon 5 of presenilin-2 mRNA by carrying out a step of contacting the HMG-I protein and the nucleic acid of the above (A), more concretely, a nucleic acid having the nucleotide sequence shown in SEQ ID NO: 4, in the presence of a substance to be tested. In addition, the binding affinity is examined for the above substance to be tested. The above binding and binding strength (for instance, binding affinity or the like) can be evaluated by a conventional technique used in analysis of protein-nucleic acid interactions, such as Southwestern analysis, gel shift assay, or resonance plasma interaction analysis.

Concretely, the above binding and the binding strength (for instance, binding affinity and the like) can be determined by, for instance, the above resonance plasma interaction analysis or the like, by using a matrix to which the nucleic acid of the above (A), more concretely a nucleic acid having the nucleotide sequence shown in SEQ ID NO: 4, or the HMG-I protein is immobilized, in the presence of a substance to be tested, to contact the matrix with a substance corresponding to the substance immobilized to the matrix, and using change in mass, change in fluorescence or the like as an index.

For instance, the above HMG-I protein can be expressed in a cell by culturing an appropriate cell under normoxia conditions and thereafter culturing the cell under hypoxic conditions.

As the normoxia conditions, the conditions can be appropriately set depending on cells used. For instance, in a case of SK-N-SH cells, the normoxia conditions include conditions such as culturing cells at 37°C in 5% CO₂. The hypoxic conditions include, for instance, those of hypoxic pressure (8 torr). Concretely, a hypoxic stimulation can be applied to the cells by maintaining the culture under hypoxic conditions at 37°C for 21 hours in an incubator equipped with a hypoxic chamber kept under a humidified atmosphere at a hypoxic pressure (8 torr).

The above cells include, for instance, cells in the central nervous system such as nerve cells, glial cells and astral cells, fibroblasts and the like, and more concretely SK-N-SH cells (ATCC HTB-11) and the like.

In addition, the protein may be purified by a conventional protein purification technique [salting-out, ion-exchange chromatography, affinity chromatography, adsorption column chromatography or the like] as occasion demands.

In the inhibitor of the present invention, when the active ingredient is a nucleic acid or a nucleic acid derivative, the nucleic acid or nucleic acid derivative may be properly carried by a vector, liposome, metal particles, a positively charged polymer or the like, which is suitable for introduction into cells in accordance with the purpose of use.

The above vector includes, for instance, nontoxic herpes virus vector, adenovirus vector, adeno-associated virus vector, nontoxic retrovirus vector, and the like.

The above liposome includes HVJ-liposomes, positively charged liposomes, and the like.

The amount of the active ingredient in the inhibitor of the present invention can be appropriately set within a range in which an action for inhibiting a binding is exhibited in accordance with the purpose of use or the like. For instance, it is desired that the active ingredient is 0.01 to 100 mg, preferably 3 to 100 mg.

The inhibitor of the present invention can suppress an aberrant splicing that lacks exon 5 of presenilin-2, whereby neuronal death can be suppressed. Therefore, according to the inhibitor, there can be provided an agent for suppressing neuronal death. The agent for suppressing neuronal death also is encompassed within the scope of the present invention.

One of the significant features of the agent for suppressing neuronal death of the present invention resides in that the agent for suppressing neuronal death comprises the above inhibitor as an active ingredient. Therefore, there is exhibited an excellent effect that neuronal death can be suppressed.

The content of the active ingredient in the agent for suppressing neuronal death of the present invention can be appropriately set depending upon body weight and age of an individual to be administered and the purpose of administration.

In addition, in the agent for suppressing neuronal death of the present invention, the dose can be appropriately selected depending upon the purpose of administration (for instance, the kind of a disease), body weight and age of an individual to be administered, the kind of an active ingredient and the like. In a case where the agent comprises, for instance, the RNA molecule, it is desired that the above dose, for instance, is such that the active ingredient is 0.01 to 100 mg, preferably 3 to 100 mg.

The method of administering an agent for suppressing neuronal death of the present invention includes *in vivo* method for directly introducing the agent into a body, and the like.

When administered by the above *in vivo* method, the administration manner is appropriately selected depending on the purpose of administration (for instance, the kind of a disease), age and body weight of an individual to be administered, the kind of the active ingredient and the like. The administration manner includes, for instance, intravenous injection, inhalation via the nasal mucosa, and the like.

For instance, when the agent for suppressing neuronal death comprising as an active ingredient an inhibitor comprising the nucleic acid or nucleic acid derivative is administered into blood, it is desired that the amount of the active ingredient is generally 0.01 to 100 mg, preferably 3 to 100 mg per day.

In addition, the agent for suppressing neuronal death of the present invention may comprise a substance capable of facilitating introduction of the agent into a nerve cell or the like, a usual carrier, excipient, binder, stabilizer, buffer, solubilizing aid, isotonic agent or the like, which is pharmacologically acceptable.

The pharmacological evaluation of the agent for suppressing neuronal death of the present invention, together with the pharmacological evaluation of the above inhibitor, can be carried out by the technique shown as follows. Concretely, the pharmacological evaluation can be carried out by examining neuronal death induced by each of hypoxic conditions [at hypoxic pressure (8 torr)] or tunicamycin or the like for SK-N-SH cells [cells to be tested] with introduction of a substance to be tested (candidate for the agent for suppressing neuronal death) and SK-N-SH cells [control cells] without the introduction. Here, a case where the neuronal death in the cells to be tested is more suppressed than the neuronal death in the control cells is used as an index showing that the substance to be tested is an agent for suppressing neuronal death. Alternatively, the pharmacological evaluation can be carried out by examining neuronal death induced by tunicamycin or the like for SK-N-SH cells [cells to be tested] transiently expressing HMG-I with introduction of a substance to be tested (candidate for the agent for suppressing neuronal death) and SK-N-SH cells [control cells] transiently expressing HMG-I. Here, a case where the neuronal death in the cells to be tested is more suppressed than the neuronal death in the control cells is used as an index that the above substance to be tested is an agent for suppressing neuronal death.

The suppression of neuronal death may be confirmed by the MTT method [Mossman, T. et al., *J. Immunol. Methods*, **65**, 55-59 (1985)], or the suppression may be confirmed by determining a viability ratio of the cells by a conventional 3-(4,5-dimethylthiazol-2-yl)-5-(3-carboxymethoxyphenyl)-2-(4-sulfonyl)-2H-tetrazolium assay (MTS assay), and comparing the viability ratio of the cells to be tested to that of the control cells.

Concretely, the above hypoxic conditions include, for instance, those conditions of applying hypoxic stimulation to the cells by exposing a culture at 37°C under low oxygen for 21 hours in an incubator equipped with a hypoxic chamber maintained, for instance, under a humidified atmosphere at hypoxic pressure (8 torr).

The inhibitor and agent for suppressing neuronal death of the present invention can suppress neuronal death, so that an aberrant splicing of presenilin-2 mRNA, particularly an aberrant splicing that lacks exon 5 of presenilin-2 can be suppressed. Therefore, according to the inhibitor and agent for suppressing neuronal death of the present invention, there can be provided a pharmaceutical composition useful for treatment or prevention of a disease caused by generation of a splicing variant that lacks exon 5 of presenilin-2 mRNA, particularly a brain disorder or neurodegenerative disease. The pharmaceutical composition is also encompassed in the present invention. Also, the inhibitor and agent for suppressing neuronal death of the present invention can be used in the manufacture of a pharmaceutical composition usable for treatment or prevention of a disease caused by generation of a splicing variant that lacks exon 5 of presenilin-2 mRNA, particularly a brain disorder or neurodegenerative disease.

One of the features of the pharmaceutical composition of the present invention resides in that the pharmaceutical composition comprises the above inhibitor or the above agent for suppressing neuronal death as an active ingredient. The pharmaceutical composition of the present invention has an action of suppressing an aberrant splicing, an action of suppressing generation of a splicing variant that lacks exon 5 of presenilin-2 mRNA, and the like. Therefore, it is expected that the pharmaceutical composition of the present invention is applied to a treatment or prevention of a disease caused by generation of a splicing variant that lacks exon 5 of presenilin-2 mRNA, particularly a brain disorder or neurodegenerative disease.

The disease to which the pharmaceutical composition of the present invention is applied includes, for instance, a brain disorder caused by generation of a splicing variant that lacks exon 5 of presenilin-2 mRNA in brain tissues, a neurodegenerative disease caused by generation of a splicing variant that lacks exon 5 of presenilin-2 mRNA, and the like.

The above brain disorder includes ischemic brain disorders, brain neurodegenerative diseases, and the like. More concretely, there are included cerebrovascular dementia, the Parkinson's syndrome, Alzheimer disease, and the like.

The above neurodegenerative disease includes amyotrophic lateral sclerosis and the like.

The dose of the pharmaceutical composition of the present invention is properly selected depending on the purpose of administration (for instance, the kind of the disease), age and body weight of an individual to be administered, the kind of the active ingredient, and the like. For instance, when the pharmaceutical composition comprises the RNA molecule, it is desired that the above dose of the active ingredient is 0.01 to 100 mg, preferably 3 to 100 mg.

The pharmaceutical composition of the present invention may be in such a form so that the active ingredient is incorporated into cells of an affected part or cells in an desired tissue. The pharmaceutical composition may be administered parenterally or topically, alone or in admixture with a conventional carrier.

The administration manner can be appropriately selected depending on the purpose of administration (for instance, the kind of a disease), age and body weight of an individual to be administered, the kind of the active ingredient, and the like. The administration manner includes intravenous injection, inhalation via the nasal mucosa, and the like.

When the pharmaceutical composition comprises, for instance, the nucleic acid or nucleic acid derivative, and is administered into blood, the pharmaceutical composition can be administered generally 3 to 100 mg per day as the amount of the active ingredient, once to several times a day.

In addition, the pharmaceutical composition can be administered continuously and gradually into an affected part by manufacturing a sustained-release preparation (mini-pellet preparation or the like), and embedding the preparation in the surroundings of the affected part, or by using an osmotic pump or the like, in order that the gene can easily exist in the surroundings of the affected site.

The pharmaceutical composition of the present invention may further comprise a substance capable of facilitating introduction of the composition into an affected site, various kinds of carriers, aids, stabilizers, lubricants and other generally usable additives, including, for instance, lactose, citric acid, tartaric acid, stearic acid, magnesium stearate, kaolin, sucrose, corn starch, talc, gelatin, agar, pectin, peanut oil, olive oil, cacao butter, ethylene glycol or the like, as occasion demands.

The pharmacological evaluation of the pharmaceutical composition of the present invention can be carried out by the same evaluation method as those in a case of the above inhibitor and the neuronal death. In addition, the conventional pharmacological evaluation can also be carried out in accordance with the subject disease.

The inhibitor and agent for suppressing neuronal death of the present invention can suppress the neuronal death, so that an aberrant splicing of presenilin-2 mRNA, particularly an aberrant splicing that lacks exon 5 of presenilin-2 can be suppressed. Therefore, according to the inhibitor and agent for suppressing neuronal death of the present invention, there can be provided a method of treating or preventing a disease caused by generation of a splicing variant that lacks exon 5 of presenilin-2 mRNA, for instance, a brain disorder or neurodegenerative disease. The method of treatment or prevention is also encompassed within the scope of the present invention.

One of the features of the method of treatment or prevention of the present invention resides in that the method comprises administering the inhibitor of the present invention or agent for suppressing neuronal death of the present invention in a therapeutically effective amount to an individual with a disease caused by generation of a splicing variant that lacks exon 5 of presenilin-2 mRNA, for instance, a brain disorder or neurodegenerative disease. According to the method of treatment or prevention of the present invention, since the above inhibitor or agent for suppressing neuronal death is administered, there is expected to treat or prevent a disease caused by generation of a splicing variant that lacks exon 5 of presenilin-2 mRNA.

Also, according to the method of treatment or prevention of the present invention, since the above inhibitor or agent for suppressing neuronal death is administered, there is exhibited an excellent effect that the action for suppressing neuronal death can be exhibited even more efficiently and even more sustainedly. Therefore, there is exhibited an excellent effect that the treatment or prevention of a disease caused by generation of a splicing variant that lacks exon 5 of presenilin-2 mRNA can be carried out even more efficiently and even more sustainedly. In addition, according to the method of treatment or prevention of the present invention, since the above inhibitor or agent for suppressing neuronal death is administered, there is exhibited an excellent effect that an RNA binding site is inhibited without impairing the function inherently owned by HMG-I exhibited upon binding of HMG-I to DNA via a DNA binding site. Therefore, according to the method of treatment or prevention of the present invention, there is exhibited an excellent effect that influences due to loss of the inherently owned functions on the basis of the binding of HMG-I to the DNA are not generated.

The "therapeutically effective amount" means an amount that can suppress neuronal death, so that an aberrant splicing of presenilin-2 mRNA, particularly an aberrant splicing that lacks exon 5 of presenilin-2, can be suppressed.

The administration of the inhibitor or agent for suppressing neuronal death to an individual can be carried out, for instance, by administering 3 to 100 mg per day as the amount of the active ingredient of the inhibitor or agent for suppressing neuronal death once to several times a day by intravenous injection, inhalation via the nasal mucosa or the like.

The effects of the method of treatment or prevention of the present invention can be evaluated by using as an index suppression of exhibition of pathological characteristics and the like of the subject disease. For instance, the effects of the method of treatment or prevention of the present invention can be evaluated by using a model with brain ischemia which includes, for instance, but is not particularly limited to, a model animal with tardive neuronal death obtained from an animal congenitally having an incomplete Willis artery ring by transient occlusion of the whole carotid arteries in both sides and subsequent re-penetration thereof or a model animal with a topical brain ischemia obtained by permanent or transient occlusion of the middle cerebral artery; administering the inhibitor or agent for suppressing neuronal death to the model; and examining the amelioration of symptoms, and the like. Further, the effects of the method of treatment or prevention of the present invention can be evaluated by administering the inhibitor or agent for suppressing neuronal death to a model animal such as a chemical hypoxia model animal obtained by applying a load to a mouse with aluminum or iron for a long duration of time or an aged mouse reflecting microtubular infarct hypoxia caused by aging, and examining the amelioration of symptoms, and the like.

In addition, the inhibitor and agent for suppressing neuronal death of the present invention can suppress the neuronal death, so that an aberrant splicing of presenilin-2 mRNA, particularly an aberrant splicing that lacks exon 5 of presenilin-2 can be suppressed. Therefore, according to the inhibitor and agent for suppressing neuronal death of the present invention, there can be manufactured a pharmaceutical composition for administering to an individual with a disease caused by generation of a splicing variant that lacks exon 5 of presenilin-2 mRNA, thereby treating or preventing the disease caused by generation of a splicing variant that lacks exon 5 of presenilin-2 mRNA. Therefore, there is encompassed in the present invention use of the inhibitor and agent for suppressing neuronal death of the present invention for manufacture of a pharmaceutical composition for administering to an individual with a disease caused by generation of a splicing variant that lacks exon 5 of presenilin-2 mRNA, thereby treating or preventing the disease caused by generation of a splicing variant that lacks exon 5 of presenilin-2 mRNA.

### Experimental Example 1: Cell culture, hypoxic stimulation and transient transfection

According to a literature by Sato et al. [Sato N. et al., *J. Biol. Chem*., **276**, 2108-2114 (2001)], cell culture and hypoxic stimulation were carried out in the following manner.

Human neuroblastoma SK-N-SH cells were cultured in 5% CO₂ at 37°C in αMEM (manufactured by GIBCO BRL) containing 10% fetal bovine serum. When the above cells achieved to confluent in a 176.6 cm² culture plate, the serum-containing α-MEM in the culture was exchanged with serum-free αMEM. The culture obtained after medium exchange was further cultured for 4 hours.

HEK-293T Cells and HeLa cells were cultured respectively in 5% CO₂ at 37°C in Dulbecco's minimum essential medium (manufactured by GIBCO BRL) containing 10% fetal bovine serum. When each of the above cells achieved to confluent in a 176.6 cm² culture plate, the serum-containing Dulbecco's minimum essential medium in the culture was exchanged with a serum-free Dulbecco's minimum essential medium. The culture obtained after medium exchange was further cultured for 4 hours.

In the case of hypoxic stimulation, the resulting culture was exposed to hypoxia at 37°C for 21 hours in an incubator equipped with a hypoxic chamber maintained in a humidified atmosphere with a low oxygen pressure (8 torr).

Transient transfection of various constructs was carried out by using LepofectAMINE™ 2000 (manufactured by GIBCO BRL).

In addition, transient transfection of an oligo RNA was carried out by using OligofectAmine (manufactured by GIBCO BRL).

### Experimental Example 2: Preparation of total RNA and RT-PCR

According to a literature by Sato et al. [Sato N. et al., *J. Neurochem*., **72**, 2498-2505 (1999)], preparations of total RNAs from neuroblastoma SK-N-SH cells, HEK-293T cells and HeLa cells under various stresses, and RT-PCR were carried out in the following manner.

Total RNAs were extracted and purified from SK-N-SH cells and HEK-293T cells under normoxia conditions, upon exposure to hypoxia, or at the time of overexpression of HMG-I, by using RNeasy total RNA kit (manufactured by Qiagen) according to manufacture's instructions.

Then, the resulting total RNA and a mouse molony leukemia virus reverse transcriptase (manufactured by Promega) were used, to perform reverse transcription at 42°C for 1 hour. Subsequently, using the resulting reaction product as a template, nested PCR was carried out. A thermal profile of PCR is 30 cycles each consisting of reactions at 95°C for 30 seconds, 60°C for 30 seconds and 72°C for 1 minute (2 minutes in the final cycle). In the 1st PCR, primer ps251 (5'-attcagacctctctgcggcc-3' [SEQ ID NO: 9]) and primer ps231 (5'-aagcgggagccaaagtctgg-3' [SEQ ID NO: 10]). In the 2nd PCR, primer ps252 (5'-gttcgtggtgcttccagagg-3' [SEQ ID NO: 11]) and primer ps233 (5'-ggaccactctgggaggtaca-3' [SEQ ID NO: 12]) were used.

The resulting product was electrophoresed on 5% polyacrylamide gel and visualized by staining with ethidium bromide.

### Experimental Example 3: Preparation of nuclear extract

According to a modified method of a method by Shreiber et al. [Yoneda, Y. et al., *Neuroscience*, **90**, 519-533 (1999)], a nuclear extract was prepared in the following manner.

All of the buffer and other solutions used were sterilized each time prior to use by filtration with Steritop (manufactured by Millipore) having a pore size of 220 nm.

Unless otherwise specified, each cell construct was homogenized at 2°C in 50 volumes [315 µl/plate] of 10 mM HEPES-NaOH buffer (pH 7.9) containing 10 mM KCI, 1 mM EDTA, 1 mM EGTA, 5 mM dithiothreitol (DTT) and 1 mM (p-amidinophenyl)methanesulfonyl fluoride (PMSF).

Then, 10% Nonidet P-40 was added at a final concentration of 0.6% to the resulting homogenate. The resulting mixture was centrifuged at 15000 rpm for 5 minutes. Then, the resulting pellet was suspended in 10 volumes [0.1 ml] of 20 mM Tris-HCl (pH 7.5) containing 400 mM KCl, 1 mM EDTA, 1 mM EGTA, 1 mM DTT and 1 mM PMSF, and then cooled on ice for 30 minutes. The suspension obtained after cooling on ice was centrifuged at 15000 rpm for 5 minutes. The resulting supernatant was used as a nuclear extract for pre-mRNA binding assay, gel shift assay and supershift assay. The above nuclear extract was stored at -80°C before use.

### Experimental Example 4: Preparation of cytosol fraction and nuclear fraction

According to a literature by Manabe et al. [Manabe et al., *Neuroscience*, **100**, 453-463 (2000)], a nuclear fraction was prepared in the following manner.

A brain structure was homogenized at 2°C in 10 mM HEPES-NaOH buffer (pH 7.9) [315 µl/plate] containing 10 mM KCI, 1 mM EDTA, 1 mM EGTA, 5 mM DTT and 1 mM PMSF. 10% Nonidet P-40 was added to the resulting homogenate so that the final concentration was adjusted to be 0.6%. The resulting mixture was centrifuged at 15000 rpm for 5 minutes, to give a pellet [nuclear fraction (insoluble in NP-40)] and a supernatant [cytosol fraction (soluble in NP-40)]. The above pellet was suspended in 50 mM Tris-HCl (pH 7.5) [50 µl] containing 1 mM EDTA, 1 mM EGTA and PMSF, to give a nuclear fraction (insoluble in NP-40).

### Experimental Example 5: Preparation of DNA constructs

The respective oligonucleotides shown in Fig. 23 was synthesized according to a conventional method. Two complementary oligonucleotides each having a nucleotide length of a 24-mer or 60-mer were used in annealing, to give double-stranded DNA fragments of Nos. 1 to 11 (SEQ ID NOs: 13 to 23). In the Figure, each fragment of PS2 exon 5 is underlined and shown in bold letters in each oligonucleotide.

Each of the resulting double-stranded DNA fragments was incorporated into pcDNA 3 (+) vector, to give DNA constructs. Each of the above double-stranded DNA fragments was sequenced by using a DNA sequencer type 373A (manufactured by Applied Biosystem).

### Experimental Example 6: Preparation of pre-mRNA probes

The DNA constructs obtained in Experimental Example 5 were linearized with *Eco*RI, to give template DNAs. The above template DNAs were then subjected to *in vitro* transcription by incubation at 37°C for 1 hour in a transcription buffer containing [α-³⁵S]-labeled UTP (62.5 µCi) or [α-³²P]-labeled UTP (50 µCi), 0.25 mM UTP, 0.5 mM ATP, 0.5 mM CTP, 0.5 mM GTP, a T7 RNA polymerase buffer, 20 U of T7 RNA polymerase (manufactured by Promega) and 40 U of RNase inhibitor (manufactured by Toyobo), to give pre-mRNA probes (RNA probes of Nos. 1 to 11).

### Experimental Example 7: Pre-mRNA binding assays

The nuclear extract of an amount equivalent to 5 µg of protein was incubated with 10 µg tRNA and each of ³⁵S-labeled RNA probes (1 µg) at 25°C for 30 minutes in an incubation buffer (12 mM HEPES-NaOH buffer [pH 7.9] containing 60 mM KCI, 4 mM MgCl₂, 1 mM EDTA, 1 mM EGTA, 1 mM DTT, 10% glycerol and 1 mM PMSF) (total volume 25 µl).

Then, the resulting reaction product was irradiated with ultraviolet (UV) rays for 15 minutes at room temperature under an UV irradiation equipment (254 nm, 60 W). Thereafter, 10 µg RNase A was added to the reaction product after irradiation with UV rays, and then incubated at 25°C for 30 minutes, to digest the probe in the above reaction product.

The resulting product was treated with sodium dodecyl sulfate (SDS) by mixing it in a volume ratio of 1 : 4 with 10 mM Tris-HCl buffer (pH 6.8) containing 10% glycerol, 2% SDS, 0.01% Bromophenol Blue and 5% 2-mercaptoethanol. The solution treated with SDS was subjected to electrophoresis on 10 to 20% gradient polyacrylamide gel or 15% polyacrylamide gel each containing 0.1% SDS at a constant current of 15 mA/plate for 2 hours at room temperature. The gel obtained after the electrophoresis was fixed, dried and exposed to an imaging plate.

### Experimental Example 8: Northern blot assays

According to a method described in the above literature by Sato et al. [Sato N. et al., *J. Biol. Chem*., **276**, 2108-2114 (2001)], Northern blot assays were carried out in the following manner. Specifically, an amount equivalent to 20 µg of total RNA was electrophoresed on formaldehyde-formamide gel, and the resulting gel was blotted onto a nylon filter. Then, the RNA transferred onto the above nylon filter was hybridized with labeled human HMG-I cDNA. The above labeled human HMG-I cDNA was prepared by labeling with Random-Labeling kit (manufactured by Takara Shuzo).

### Experimental Example 9: Immunoblot assays

According to the above literature by Manabe et al. [Manabe et al., *Neuroscience*, **100**, 453-463 (2000)], immunoblot assays were carried out in the following manner. Specifically, the amount of proteins in each of the nuclear extract, the whole lysate, the cytosol fraction and the nuclear fraction was measured. Then, an aliquot of each of the nuclear extract, the whole lysate, the cytosol fraction and the nuclear fraction was mixed in a volume ratio of 1 : 4 with a buffer [10 mM Tris-HCl, 10% glycerol, 2% sodium dodecyl sulfate (SDS), 0.01% Bromophenol Blue, 5% mercaptoethanol, (pH 6.8)]. The resulting mixture was boiled at 100°C for 10 minutes. A given amount of each aliquot (an amount equivalent to 25 µg of protein for the nuclear extract or 50 µg of protein for the whole lysate, cytosol fraction or nuclear fraction) was electrophoresed on 15% polyacrylamide gel containing 0.1% SDS at a constant current of 15 mA/plate for 2 hours at room temperature. Each gel after electrophoresis was blotted onto a polyvinylidene difluoride (PVDF) membrane activated with 100% methanol. The PVDF membrane obtained after blotting was blocked with PBS containing 0.05% Tween-20 and 5% skim milk. Then, the PVDF membrane was reacted with an anti-HMG-I/Y antibody diluted with PBS containing 0.05% Tween-20 in which 1% skim milk was contained. The PVDF membrane was then reacted with an alkaline phosphatase-conjugated anti-goat immunoglobulin G (IgG) antibody. The immunoreactivity was visualized in 100 mM Tris-HCl buffer (pH 9.5) containing 100 mM NaCl, 5 mM MgCl₂, 66.7% 4-nitro blue tetrazolium chloride (NTB) and 33.3% X-phosphate/5-bromo-4-chloro-3-indolyl phosphate (BCIP).

### Experimental Example 10: Immunoprecipitation

According to a method described in the above literature by Sato et al. [Sato N. et al., *J. Biol. Chem*., **276**, 2108-2114 (2001)], immunoprecipitation was carried out in the following manner.

The nuclear extract derived from SK-N-SH cells under normoxia or the nuclear extract derived from SK-N-SH cells exposed to hypoxia was incubated at 4°C for 8 hours with an anti-HMG-I antibody (manufactured by Santa Cruz Biotechnology). The resulting product was mixed with protein-G agarose (manufactured by Pharmacia Biotech) and kept at 4°C for 1 hour. The resulting mixture was centrifuged at 3000 rpm for 5 minutes, and the resulting precipitates were suspended in PBS. Further, the resulting suspension was centrifuged at 3000 rpm for 5 minutes. The resulting precipitates were suspended in Mili-Q water. This suspension was treated with SDS, and the resulting solution was subjected to SDS-PAGE. The resulting gel was subjected to immunoblot assay using an antibody against U1 (70 K) snRNP.

Also, the nuclear extracts were subjected to immunoprecipitation using an anti-U1 (70 K) snRNP antibody (manufactured by Santa Cruz Biotechnology) in place of the above anti-HMG-I antibody, and then subjected to immunoblot assay using an antibody against HMG-I/Y (anti-HMG-I/Y antibody, manufactured by Santa Cruz Biotechnology) in place of the above antibody against U1 (70 K) snRNP.

### Experimental Example 11: Immunocytochemistry

The immunocytochemistry of SK-N-SH cells was carried out. Specifically, SK-N-SH cells were maintained under normoxia conditions, or the cells were exposed to hypoxic conditions for 21 hours. Then, the resulting cells were fixed in 4% paraformaldehyde at room temperature for 2 hours and permeated in 0.3% Triton-X100 for at least 5 minutes. The fixed and permeated cells were washed 3 times with PBS. The washed cells were subjected to immunoreaction using an anti-HMG-I/Y antibody and anti-SC35 antibody (manufactured by Sigma) at 4°C for 12 hours. The cells were washed 3 times with PBS and incubated with FITC-conjugated anti-goat IgG antibody and Cy3-conjugated anti-mouse IgG antibody at room temperature for 2 hours. The immunoreactive protein was observed with a 488 nm excitation filter under a microscope equipped with a computer.

### Experimental Example 12: Immunohistochemistry

An immunohistochemical method was carried out by using a modified immunoperoxidase method described in the above literature by Sato et al. [Sato N. et al., *J. Biol. Chem*., **276**, 2108-2114 (2001)]. Specifically, a control or an sAD brain section was fixed at room temperature for 2 hours, and then treated for HMG-I/Y immunohistochemistry. The anti-HMG-I/Y antibody or anti-SS MAG antibody was used at a dilution of 1 : 1000. A biotinylated anti-goat antibody or biotinylated anti-rabbit IgG (manufactured by Vectastain Elite) was used as a secondary antibody. Immunoreactivity was visualized with 0.05% DAB and 0.01% hydrogen peroxide in 50 mM Tris (pH 7.6).

### Experimental Example 13: Super shift assay

A nuclear extract of an amount equivalent to 5 µg of protein was pretreated by incubation at 4°C for 8 hours with 1 µg anti-HMG-I/Y antibody (manufactured by Santa Cruz Biotechnology) or 0.5 to 2.0 µg anti-U1 (70 K) snRNP antibody (manufactured by Santa Cruz Biotechnology). The resulting pretreated product was incubated with 2 µg or 10 µg tRNA in an incubation buffer [composition: 12 mM HEPES-NaOH buffer (pH 7.9) containing 60 mM KCI, 4 mM MgCl₂, 1 mM EDTA, 1 mM EGTA, 1 mM DTT, 10% glycerol and 1 mM PMSF]. Then, ³²P-labeled RNA probe [1 µg, the probe of No. 5 shown in Fig. 4] was added thereto and incubated at 25°C for 30 minutes (total volume 50 µl).

The resulting reaction product was electrophoresed at a constant voltage of 11 V/cm for 1.5 hours at 4°C on 4% polyacrylamide gel in a buffer (pH 8.5) containing 50 mM Tris, 0.38 M glycine and 2 mM EDTA, whereby the bound probe was separated from the free probe. After electrophoresis, the gel was fixed, dried and then exposed to an X-ray film.

### Example 1: Expression of PS2V in brain of patient with sporadic Alzheimer's disease and in cultured cells

### (1) Expression of PS2V in brain of patient with sporadic Alzheimer's disease

The total RNA derived from the brain of a patient with sporadic Alzheimer's disease (sAD) or the total RNA derived from a control brain of an age-matched patient was assayed by RT-PCR.

According to a method described by Anwar R. et al. (Anwar R. et al., *J. Neurochem*., **66**, 1774-1777 (1996)), mRNA was isolated from the sAD brain and the control brain.

Then, the resulting mRNA and a mouse molony leukemia virus reverse transcriptase (manufactured by Promega) were used, to perform reverse transcription reaction. The thermal profile of PCR is 30 cycles, wherein one cycle consists of denaturation: 95°C, 2 minutes/95°C, 30 seconds, annealing: 60°C, 30 seconds and extension: 72°C, 1 minute. In addition, as the 1st primers, the above primers ps251 and ps231 were used, and as the 2nd primers, the primers ps252 and ps233 were used.

The resulting reaction product was electrophoresed on 5% polyacrylamide gel. The amplified product was visualized by staining with ethidium bromide. The results are shown in Fig. 1.

As shown in lane 1 in the left panel in Fig. 1 and in the literature by Sato et al. [Sato et al., *J. Neurochem*., **72**, 2498-2505 (1999)], the full-length PS2 can be detected as the major RT-PCR product in the control brain. On the other hand, as shown in lane 2 in the left panel in Fig. 1, a shorter chain product, in addition to the full-length PS2, can be detected in the sAD brain. As a result of direct sequencing, it is revealed that the above shorter chain product is a variant that lacks exon 5 of PS2 gene (hereinafter, referred to as PS2V).

### (2) Expression of PS2V in cultured cells

For efficiently reproducing the production of PS2V in cultured cells as in the sAD model, total RNAs derived from various cell lines under various stresses were determined for expression of PS2V in the same experiment as mentioned above. The results are shown in the right panel in Fig. 1.

As shown in lanes 1 to 3 in the right panel in Fig. 1, only the full-length PS2 is detected in various cell lines, namely, HeLa cells, HEK-293T cells and SK-N-SH cells which are exposed to normoxia conditions, respectively. Also, as shown in lanes 4 and 5 in the right panel in Fig. 1, the same results are shown in HeLa cells and HEK-293T cells exposed to hypoxic conditions.

However, it is found that PS2, and a short chain product confirmed to be PS2V by direct sequencing, are detected in SK-N-SH cells exposed to hypoxic conditions [lane 6 in the right panel in Fig. 1; Sato et al., *J. Neurochem*., **72**, 2498-2505 (1999)] and in the sAD brain.

On the other hand, detectable PS2V was not observed under other stresses, namely, tunicamycin (lane 7 in the right panel in Fig. 1), β-amyloid (lane 8 in the right panel in Fig. 1) and hydrogen peroxide [Sato et al., *J. Neurochem*., **72**, 2498-2505 (1999)].

Then, the localization of PS2V was examined by immunohistochemical staining using an antibody against PS2V protein (anti-SSMAG antibody). The results are shown in Fig. 2.

As shown in Fig. 2 and in the literature by Sato et al. [Sato N. et al., *J. Biol. Chem*., **276**, 2108-2114 (2001)], the immunoreactivity to PS2V is observed in a pyramidal cell layer in a hippocampus CA1 region and a pyramidal cell layer in a temporal cortex in sAD brain tissues. Accordingly, it is found that PS2V is localized in a pyramidal cell layer in a hippocampus CA1 region and a pyramidal cell layer in a temporal cortex in sAD brain tissues.

### Example 2: Detection of PS2 pre-mRNA-binding factor in hypoxia-exposed cells

In order to detect a PS2 pre-mRNA-binding factor in hypoxia-exposed cells, various ³⁵S-labeled pre-mRNA probes (RNA probes) shown in Fig. 4 were prepared. By the strategy shown in Fig. 3, a unique pre-mRNA binding assay using the probes as shown in Fig. 4 was established. The results are shown in Fig. 5.

As a result of the pre-mRNA binding assay using ³⁵S-labeled probe of No. 0, a signal was not detected at the position of the molecular weight (about 20 kDa) indicated by the black arrow, in the nuclear extract derived from the neuroblastoma SK-N-SH cells under normoxia conditions, as shown in lane 1 in Fig. 5, and in the nuclear extract derived from the neuroblastoma SK-N-SH cells exposed to hypoxic conditions for 21 hours, a signal was detected at the position of the molecular weight (about 20 kDa) indicated by the black arrow, as shown in lane 2 in Fig. 5. Accordingly, it is found that a factor exhibiting PS2 pre-mRNA binding activity is present in the nuclear extract derived from hypoxia-exposed neuroblastoma SK-N-SH cells. By heating the nuclear extract prior to use in pre-mRNA binding assay, the PS2 pre-mRNA binding activity almost disappears, and thus there is a high possibility that this binding activity is derived from a protein but not derived from other factors such as nucleic acid and lipid.

In addition, as shown in lanes 3 to 12 in Fig. 5, it is found that the factor exhibiting PS2 pre-mRNA binding activity, found in the nuclear extract derived from the SK-N-SH cells exposed to hypoxic conditions, binds to the 3'-site of PS2 exon 5.

Further, the influence of each of unlabeled probes of Nos. 1 to 5 on the binding between the probe of No. 0 and the above factor found in the nuclear extract derived from the SK-N-SH cells exposed to hypoxic conditions was examined by pre-mRNA binding assays. The results are shown in Fig. 6.

As shown in Fig. 6, it is found that the binding between the probe of No. 0 and the above factor found in the nuclear extract derived from the hypoxia- exposed SK-N-SH cells is inhibited by addition of ³⁵S-unlabeled the probe of No. 5, but not inhibited by the other unlabeled probes (Nos. 1 to 4).

Further, the influence of normoxia conditions and hypoxia on induction of the binding activity to the probe of No. 5 in the nuclear extract obtained at different stages after stimulation was examined. As a result, a marked binding activity to the probe of No. 5 under the conditions used was not noted in the nuclear extract derived from the SK-N-SH cells exposed to normoxia conditions. However, the enhancement of the binding activity by hypoxia was noted in the nuclear extract between 16 and 21 hours after stimulation, and persisted for at least 24 hours.

All the experiments in this example were repeated at least 4 times by using other cell cultures, and the same results were obtained. Accordingly, it is suggested that the binding activity induced by hypoxia is specific to the probe of No. 5.

### Example 3: Purification and identification of human PS2 exon 5-binding protein

To purify the factor having a binding activity to the probe of No. 5 in Example 2, the fraction to be tested was assayed by adding it to the pre-mRNA-binding reaction product and measuring an increase in the binding activity to the probe of No. 5.

Scheme of purification procedures are shown in Fig. 7. The respective fractions (I) to (V) shown in Fig. 7 were assayed by silver staining (Fig. 8) and RNA binding assays (Fig. 9).

The nuclear extract (Fig. 9, lane 1) derived from SK-N-SH cells exposed to hypoxic conditions in the same manner as in Example 1 was fractionated with 60% saturated ammonium sulfate, to give a fraction as a supernatant having a binding activity to the RNA probe of No. 5 [fraction (II), Fig. 7 (II)]. The result of silver staining of the above fraction (II) is shown in lane 2 in Fig. 8, and the result of RNA binding assay is shown in lane 2 in Fig. 9.

The above fraction (II) was dialyzed twice against 5 L of 50 mM Tris-HCl buffer (pH 7.5) at 4°C for 4 hours, thereby removing ammonium sulfate. The resulting dialysate was subjected to anion-exchange chromatography on DEAE column (manufactured by Pharmacia Biotech), and then the column was washed with 10 ml of 50 mM Tris-HCl buffer (pH 7.5). Then, the elution was carried out by using 10 ml of 50 mM Tris-HCl buffer (pH 7.5) containing 1 M NaCl, to give a fraction [fraction (III), Fig. 7 (III)] having a potent binding activity to the RNA probe of No. 5. The result of silver staining of the above fraction (III) is shown in lane 3 in Fig. 8, and the result of RNA binding assay thereof is shown in lane 3 in Fig. 9.

The above fraction (III) was dialyzed twice against 5 L of 50 mM Tris-HCl buffer (pH 7.5) at 4°C for 4 hours. Then, the resulting dialysate was applied to 5'-amino-2'-O-methyl oligo RNA (5'-aucuucuugguggugcucuacaaguaccgcugcuacaaggugaggcccu-3', SEQ ID NO: 24)-coupled CNBr-activated Sepharose 4B (manufactured by Pharmacia Biotech) affinity column. The above affinity column was washed with 10 ml of the above incubation buffer and then eluted with 15 ml of the incubation buffer containing 1 M NaCl to give a fraction having a binding activity to the RNA probe of No. 5 [fraction (IV), Fig. 7 (IV)] (Fig. 9, lane 4). The result of silver staining of fraction (IV) is shown in lane 4 in Fig. 8, and the result of RNA binding assay thereof is shown in lane 4 in Fig. 9.

From the result of silver staining, it was found that the fraction was not completely purified, as shown in lane 4 in Fig. 8.

Then, the above fraction (IV) was dialyzed twice against 5 L of Mili-Q water at 4°C for 4 hours. The resulting dialysate was completely lyophilized and then redissolved in Mili-Q water containing 0.1% TFA.

The resulting solution was subjected to reverse-phase chromatography on Waters 5C18-MS (4.6 mm × 150 mm) column. Specifically, the column was washed with Mili-Q water containing 0.1% TFA. Thereafter, the elution was carried out by using a linear gradient of 0 to 100% acetonitrile in 25 ml Mili-Q water containing 0.1% TFA, followed by 5 ml of 100% acetonitrile containing 0.1% TFA.

As a result, a fraction showing a single peak eluted with 20 to 25% acetonitrile [fraction (V), Fig. 7 (V)] was obtained. This fraction showed a single band at the molecular weight indicated by the black arrow, as shown in lane 5 in Fig. 8, by silver staining after SDS-PAGE. However, the binding activity of the final fraction was dramatically reduced as compared in the previous step [Fig. 7 (IV)]. When the final concentration of acetonitrile in the binding reaction solution was adjusted to 30% by weight, the binding activity of the original fraction to the probe of No. 5 was completely lost, and thus it is found that the reduction in the binding activity of the final fraction depends on acetonitrile contained in the elution buffer for reverse-phase chromatography.

The resulting fraction was separated by electrophoresis on 12% polyacrylamide gel containing 0.1% SDS at a constant current of 15 mA/plate for 2 hours at room temperature. Thereafter, a gel section containing the separated protein band was cut out, and the protein was digested in gel with trypsin. The eluted peptide fragment was separated by HPLC with TSK gel ODS-80Ts QA column (2.0 mm × 250 mm, manufactured by Tosoh). Then, the separated peptide fragment was analyzed with an automated protein sequencer (HP G1005A Protein Sequencing System). The obtained information on the peptide sequence was used for searching data bases.

The single band in the final fraction was analyzed for its peptide sequence, to give 17 amino acid sequences. The obtained peptide sequence was compared with known proteins in a sequence data base, and as a result, PS2 pre-mRNA-binding protein in the nuclear extract derived from hypoxia-exposed SK-N-SH cells matched completely with HMG-I (GenBank Accession No. P17096).

### Example 4: Evaluation of binding activity of HMG-I

To confirm that HMG-I actually has a binding activity on the basis of the results in Example 3, an antibody against HMG-I protein and the nuclear extract derived from hypoxia-exposed SK-N-SH cells or the nuclear extract derived from SK-N-SH cells under normoxia conditions were used in supershift assays according to Experimental Example 13, as follows.

Specifically, the nuclear extract of an amount equivalent to 5 µg of protein was pretreated by incubation at 4°C for 8 hours with 0.5 to 2.0 µg anti-HMG-I/Y antibody (manufactured by Santa Cruz Biotechnology). The pretreated nuclear extract was incubated with 10 µg tRNA in the incubation buffer, and then a total volume of 50 µl of ³²P-labeled RNA probe [1 µg, the probe of No. 5 shown in Fig. 4] was added to the resulting product and incubated at 25°C for 30 minutes. The bound probe was separated from the free probe by electrophoresis at a constant voltage of 11 V/cm for 1.5 hours at 4°C on 4% polyacrylamide gel in a buffer (pH 8.5) containing 50 mM Tris, 0.38 M glycine and 2 mM EDTA. The gel was fixed and dried. Then, the dried gel was exposed to an X-ray film, thereby performing to autoradiography.

As a result, the nuclear extract derived from hypoxia-exposed SK-N-SH cells showed more potent binding activity than that of the nuclear extract derived from SK-N-SH cells under normoxia conditions, as shown in lanes 1 and 2 in the right panel in Fig. 10. In addition, it is found that by the pretreatment with the anti-HMG-I/Y antibody, this increase in the binding activity was super-shifted in an antibody concentration-dependent manner, as shown in lanes 3 to 5 in the right panel in Fig. 10. However, the nuclear extract derived from SK-N-SH cells under normoxia conditions in the control experiment did not show a significant effect attributable to addition of the HMG-I/Y antibody, as shown in the left panel in Fig. 10.

From these results, it is revealed that the substance having a binding activity to the probe of No. 5, derived from hypoxia-exposed SK-N-SH cells, is HMG-I.

Further, it is revealed that in the hypoxia-exposed cells, the binding activity of HMG-I protein to the probe of No. 5 does not depend on UV irradiation in the pre-mRNA binding assay.

### Example 5: Determination of PS2 pre-mRNA binding site

The present inventors directed to their attention to two repeated analogous sequences (repeated sequences) at the 3'-terminal of PS2 exon 5 for the probe of No. 5 in Fig. 4, and estimated that HMG-I protein bind to these repeated sequences. Accordingly, six RNA probes (the probes of Nos. 6 to 11) shown in Fig. 11 were prepared as derivatives of the probe of No. 5, and the resulting probes were used in pre-mRNA binding assays according to the above Experimental Example 7. The results are shown in Fig. 12. The above repeated sequences are the underlined sequences in the probe of No. 5 in Fig. 11, and shown in SEQ ID NOs: 1 and 2, respectively.

As shown in lane 1 in Fig. 12, the binding activity to the probe of No. 5 could not be detected in the nuclear extract derived from SK-N-SH cells exposed to normoxia conditions. On the other hand, it was found that in the nuclear extract derived from hypoxia-exposed SK-N-SH cells, the binding activity to the probe of No. 5 was strongly increased at the position of the molecular weight indicated by the black arrow, as shown in lane 2 in Fig. 12 similar to those in Figs. 5 and 6.

However, as shown in lane 3 in Fig. 12, this increase in the binding activity by exposure to hypoxia disappeared by using a probe (the probe of No. 6 in Fig. 11) lacking the sequences of SEQ ID NOs: 1 and 2. In the nuclear extract derived from hypoxia-exposed SK-N-SH cells, however, the binding was significantly increased (Fig. 12, lane 4) by using the probe of No. 7 (Fig. 11) containing SEQ ID NOs: 1 and 2 bound directly thereto. Further, when the probe of No. 8 (Fig. 11) containing those sequences of SEQ ID NOs: 1 and 2 wherein C was replaced by A, the binding activity disappeared completely in almost all the regions as shown in lane 5 in Fig. 12. Further, when the binding activity was observed by using either of SEQ ID NO: 1 or 2 in the binding assay (Fig. 12, lanes 6 and 8), the binding activity to the probe of No. 10 having both the sequences was the highest (Fig. 12, line 7). These results mean that either one or both of SEQ ID NOs: 1 and 2 are necessary for the binding activity of HMG-I protein to PS2 pre-mRNA.

Fig. 12 shows that either one or both of SEQ ID NOs: 1 and 2 are necessary for the binding activity of HMG-I to PS2 pre-mRNA. When the above sequences of SEQ ID NO: 1 and SEQ ID NO: 2 were examined for homology in comparison with other genes in data bases, it is revealed that the above SEQ ID NOs: 1 and 2 are sequences unique to PS2 exon 5.

The above results show that the HMG-I/Y protein binds to a single-stranded RNA, and the HMG-I protein recognizes and binds to a site consisting of a sequence comprising gcucuacaag (SEQ ID NO: 1) and/or gcugcuacaag (SEQ ID NO: 2).

### Example 6: Influence of hypoxia on expression of HMG-I mRNA and immunoreactive HMG-I protein

To determine whether or not the increase in the binding activity depends on an increase in expression of HMG-I, the expression levels of HMG-I mRNA and the protein under normoxia conditions was compared with those upon exposure to hypoxia.

As shown in the upper left panel in Fig. 13, it is revealed by Northern blot analysis that the HMG-I mRNA level is dramatically increased by exposure to hypoxia which is the condition for expressing PS2V in the neuroblastoma SK-N-SH cells. However, when hypoxia-exposed HEK-293T cells not inducing PS2V were used (Fig. 13, the upper middle panel) or tunicamycin-treated SK-N-SH cells not inducing PS2V were used (Fig. 13, the upper middle panel), no significant increase was observed under stress.

As a result, as indicated by the upper arrow in the left panel in Fig. 14, it is found by immunoblot analysis with the anti-HMG-I/Y antibody that the immunoreactive HMG-I protein was expressed at higher levels in the nuclear extract of hypoxia-exposed SK-N-SH cells than in the extract of the cells under normoxia conditions. However, it is found that in the nuclear extract derived from SK-N-SH cells, the immunoreactive HMGY protein does not undergo the effect of hypoxic stress, as indicated by the lower arrow in the left panel in Fig. 14. As shown in the right panel in Fig. 14, it is found that no significant immunoreactivity with anti-HMG-I/Y antibody is observed in the hypoxia-exposed HEK-293T cells.

The results shown in the experiment using hypoxia-exposed HEK-293T cells and tunicamycin-treated SK-N-SH cells are contradictory to those of a previous report showing that the cells with HMG-I/Y mRNA in a steady state are first increased due to hypoxia, resulting in an increase in HMG-I/Y protein [Ji, Y. S. et al., *Circ. Res*., **83**, 295-304 (1998)]. This evident contradiction may reflect a difference among the respective cell lines.

### Example 7: Localization of HMG-I protein at the cellular level

To examine the localization of the protein in the cells, the SK-N-SH cell culture was exposed to hypoxic conditions for 21 hours, and the HMG-I protein was detected under an immunofluorescence microscope by using an anti-HMG-I/Y antibody and anti-SC35 antibody. The above anti-SC35 antibody is an antibody against SC35 protein, a factor essential for splicing. In this example, an immunocytochemistry was carried out using the above anti-SC35 antibody as a maker for a speckle where a splicing factor is present.

It is found in the immunocytochemistry that, as shown in normoxia conditions in Fig. 15, the HMG-I protein was mainly localized in the nuclei in the cells under normoxia conditions, and weak and scattering immunoreactions were obtained in the cytoplasm without co-localizing with the immunoreactive SC35 protein occurring only in the nuclear speckle.

In the nuclear speckle where SC35 was co-localized, however, more potent immunoreactivity was obtained, and the immunoreactivity in the cytoplasm in the hypoxia-exposed SK-N-SH cells was lower than in the cells under normoxia conditions. Accordingly, the HMG-I protein is not only induced in the SK-N-SH cells by hypoxic stimulation but also accumulated from the cytoplasm to the nuclei, and there would be a correlation between expression of HMG-I and induction of PS2V.

### Example 8: In vivo effect of transient transfection with HMG-I and HMG-Y on PS2 gene exon 5 skipping

According to the above literature by Sato et al. [Sato et al., *J. Neurochem*., **72**, 2498-2505 (1999)], alternatively spliced form PS2 gene lacking exon 5 was obtained from a brain affected with sporadic Alzheimer's disease and a hypoxia-exposed SK-N-SH cell respectively.

To determine whether or not alternative splicing accompanied by lack of defection in PS2 gene exon 5 was caused by HMG-I/Y, the effect of the transient expression of HMG-I on induction of PS2V in SK-N-SH cultured cells and HEK-293T cultured cells was examined. The results of immunoblot analysis are shown in Fig. 16, and the results of RT-PCR are shown in Fig. 17.

As shown in Fig. 16, it was found that the nuclear extract derived from HMG-I-transfected SK-N-SH cells and the nuclear extract derived from HMG-I-transfected HEK-293T cells had expressed HMG-I protein having more potent immunoreactivity than in the nuclear extract derived from mock-transfected cells. Further, it is found that in RT-PCR assay of the total RNA derived from mock-transfected SK-N-SH cells, the full-length PS2 gene was detected as the major transcription product (PS2 in Fig. 17) at the position of its corresponding molecular weight, as shown in lane 1 in Fig. 17. In contrast, when the total RNA derived from HMG-I-transfected cells was used, it was found by RT-PCR assay that not only the full-length PS2 as the major transcription product (PS2 in Fig. 17) but also a shorter chain RT-PCR product (PS2V in Fig. 17) was detected in a manner which depends on the amount of HMG-I, as shown in lanes 2 and 3 in Fig. 17.

By direct sequence analysis, it is revealed that the abnormal RT-PCR product is an alternative splicing product of PS2 lacking exon 5. On the other hand, weak induction of PS2V is observed in SK-N-SH cells upon the transient transfection of HMG-Y, but the HMGY protein is not induced by hypoxia in the nuclear extract derived from SK-N-SH cells, as shown in lanes 4 and 5 in Fig. 17. Further, in the HEK-293T cell which is a cell line wherein PS2V is not induced by hypoxia (Fig. 2), PS2V is detected due to overexpression of HMG-I, as shown in Fig. 17.

It is known that U1 (70 K) snRNP, an essential factor for splicing, usually binds to a 5'-splicing site. Accordingly, it is expected that HMG-I can inhibit the binding of U1 snRNP to pre-mRNA. Hence, the effect of HMG-I on the binding activity of U1 (70K) snRNP to pre-mRNA in the nuclear extract derived from SK-N-SH cells was examined.

Using an antibody against U1 (70 K) snRNP [anti-U1 (70 K) snRNP antibody manufactured by Santa Cruz Biotechnology], supershift assay was carried out according to Experimental Example 13, as follows.

Specifically, the nuclear extract of an amount equivalent to 5 µg of protein was pretreated by incubation at 4°C for 8 hours with 1 µg anti-HMG-I/Y antibody (manufactured by Santa Cruz Biotechnology). The resulting pretreated product was incubated with 2 µg tRNA in an incubation buffer [composition: 12 mM HEPES-NaOH buffer (pH 7.9) containing 60 mM KCI, 4 mM MgCl₂, 1 mM EDTA, 1 mM EGTA, 1 mM DTT, 10% glycerol and 1 mM PMSF]. Then, ³²P-labeled RNA probe [1 µg, the probe of No. 5 shown in Fig. 4] was added thereto and incubated at 25°C for 30 minutes (total volume: 50 µl). The resulting reaction product was electrophoresed at a constant voltage of 11 V/cm for 1.5 hours at 4°C on 4% polyacrylamide gel in a buffer (pH 8.5) containing 50 mM Tris, 0.38 M glycine and 2 mM EDTA, whereby the bound probe was separated from the free probe. The gel after electrophoresis was fixed, dried and exposed to an X-ray film. The results are shown in Fig. 18.

As shown in the left panel in Fig. 18, it is found that by the pretreatment with the anti-U1 (70 K) snRNP antibody, the binding activity to the probe of No. 12 in the nuclear extract derived from Mock-transfected SK-N-SH cells was super-shifted in an antibody-concentration dependent manner. In addition, as shown in lane 1 in the left panel and lane 1 in the right panel in Fig. 18, more potent binding activity is observed in the nuclear extract derived from HMG-I-transfected SK-N-SH cells than in the nuclear extract derived from Mock-transfected cells.

However, the binding activity in the nuclear extract from HMG-I-transfected SK-N-SH cells was not affected by addition of the anti-U1 (70 K) snRNP antibody, as shown in lanes 2 to 4 in the right panel in Fig. 18. Namely, these results indicate that HMG-I inhibits the activity of U1 (70 K) snRNP to bind to pre-mRNA.

Further, the results of determining the inhibitory effect of HMG-I on the binding activity of U1 (70 K) snRNP to pre-mRNA are shown in Fig. 19.

As shown in Fig. 19, it is found that HMG-I binds directly to U1 (70 K) snRNP in the nuclear extract derived from hypoxia-exposed SK-N-SH cells, but does not bind to U1 (70 K) snRNP in the nuclear extract derived from the cells exposed to normoxia conditions.

The transient transfection of HMG-I in SK-N-SH cells induced expression of PS2V (Fig. 17). Further, detectable PS2V was observed (Fig. 17) by overexpression of HMG-I in the HEK-293T cell line which is a cell line not expressing PS2V (Fig. 2) by exposure to hypoxia. Accordingly, it can be said that PS2V is induced by merely overexpressing HMG-I in the cells. It is suggested that an increase in the expression of HMG-I is necessary rather than stimulation with hypoxia in order to induce PS2V.

U1 (70 K) snRNP is an essential factor for splicing, and recognizes a 5'-splicing site and binds to the 5'-splicing site. U1 (70 K) snRNP recognition sequence and HMG-I recognition sequence are close to each other on the probe of No. 5. The binding activity of U1 (70 K) snRNP to PS2 mRNA was inhibited *in vitro* in the nuclear extract derived from HMG-I-transfected SK-N-SH (Fig. 18).

Further, the HMG-I protein bound to U1 (70 K) snRNP in the nuclear extract derived from hypoxia-exposed SK-N-SH cells (Fig. 19). It is presumed that the association between HMG-I and U1 (70 K) snRNP observed in this example inhibits the binding of SF2/ASF to U1 (70 K) snRNP, resulting in inhibition of the binding activity of U1 (70 K) snRNP to pre-mRNA.

In addition, there is suggested a possibility that HMG-I inhibits the binding of U1 (70 K) snRNP to pre-mRNA or another splicing factor as shown in Fig. 20, thereby inhibiting the action of U1 (70 K) snRNP on PS2 pre-mRNA.

### Example 9: Expression of HMG-I/Y protein in sAD brain

It is presumed that if HMG-I protein actually induces alternative splicing of PS2 lacking exon 5, higher expression of HMG-I must be observed in a sAD brain than in a control brain. Accordingly, the expression level of HMG-I in the control brain was compared with that of the sAD brain by immunoblot assays. The results are shown in Fig. 21.

As shown in the left and right panels in Fig. 21, both HMG-I protein and HMG-Y protein are increased as compared with those in the age-matched control, and expressed in the whole lysate and nuclear fraction derived from the hippocampuses of three different sAD patients. In the cytosol fraction of hippocampus, however, there is no significant difference between the sAD patients and the control, as shown in the middle panel in Fig. 21.

To specify the HMG-I/Y-expressing region in the hippocampus, immunohistochemical analysis was carried out using an anti-HMG-I/Y antibody. The results are shown in Fig. 22. As shown in Fig. 22, a higher exhibition of immunoreactivity is recognized in a pyramidal cell layer in the hippocampus CA1 region of the sAD brain than in the control.

In immunohistochemical analysis, the immunoreactive HMG-I/Y protein was detected in the nuclear fraction derived from the hippocampus of the control brain (Fig. 21), but substantially no HMG-I/Y protein was detected in the hippocampal CA1 region of the control brain (Fig. 22). This is because HMG-I/Y was expressed in a region of hippocampal stratum lacunosum-moleculare of the control, and there was no difference as compared with the sAD brain.

More potent expression of both HMG-I protein and HMG-Y protein was observed in the nuclear fraction of the hippocampus of the sAD brain than in the control, but not observed in the cytosol fraction (Fig. 21). Also, by immunohistological assay, the HMG-I/Y protein was detected in pyramidal cells in the hippocampus CA1 region of the sAD brain (Fig. 22). These results show a possibility that the HMG-I/Y protein binds to PS2 pre-mRNA, to actually induce a splicing variant that lacks exon 5 of PS2 gene in the brain and the cell line with sAD.

### Example 10

According to the above literature by Sato et al., preparation of the total RNA of neuroblastoma SK-N-SH cells and RT-PCR were carried out, and the inhibitory effect of 2'-O-methyl oligo RNA having the nucleotide sequence shown in SEQ ID NO: 27 on generation of a splicing variant that lacks exon 5 of presenilin-2 mRNA was studied in the following manner.

Neuroblastoma SK-N-SH cells were plated in 8 ml of Dulbecco's minimum essential medium (manufactured by GIBCO BRL) containing 10% fetal bovine serum, and then transfected with 0 µg, 10 µg or 15 µg of 2'-O-methyl oligo RNA (SEQ ID NO: 27) (0 µl, 20 µl and 45 µl, respectively) using oligofectamine. When only oligofectamine (0 µl, 20 µl and 45 µl, respectively) was used, no significant change was recognized.

The above cells were cultured under the conditions of 5% CO₂ at 37°C for 16 hours. After culture for 12 hours, the medium was exchanged with another. Thereafter, the resulting cells were cultured for additional 21 hours in a hypoxia chamber (oxygen pressure, 8 torr).

Then, the total RNA was extracted and purified in the same manner as above by using RNeasy total RNA kit (manufactured by Qiagen).

Using the resulting total RNA and a mouse molony leukemia virus reverse transcriptase (manufactured by Promega), reverse transcription reaction was performed at 42°C for 1 hour. Using the resulting reaction product as a template, the 1st PCR was carried out. Using the resulting PCR product as the template, the 2nd PCR was carried out.

A thermal profile of PCR is 30 cycles, wherein one cycle consists of reactions at 95°C for 30 seconds, 60°C for 30 seconds, and 72°C for 1 minute (72°C for 2 minutes in the final cycle). In the 1st PCR, primer ps251 (SEQ ID NO: 9) and primer ps231 (SEQ ID NO: 10) were used. In the second PCR, primer ps252 (SEQ ID NO: 11) and primer ps233 (SEQ ID NO: 12) were used.

The resulting product was electrophoresed on 5% polyacrylamide gel (TBE) and visualized by staining with ethidium bromide.

As the control, RNA derived from neuroblastoma SK-N-SH cells into which 2'-O-methyl oligo RNA had not been introduced was used. The results are shown in Fig. 24.

As shown in Fig. 24, the generation of a splicing variant that lacks exon 5 of presenilin-2 mRNA (PS2V in the Figure) was more inhibited where the 2'-O-methyl oligo RNA was introduced than where it was not introduced.

### Example 11

The influence of 2'-O-methyl oligo RNA (SEQ ID NO: 27) on hypoxia-induced binding of HMG-I to PS2 pre-mRNA was studied *in vitro*.

The SK-N-SH cells were cultured in 5% CO₂ at 37°C for 21 hours under normoxia conditions or hypoxic conditions. A nuclear extract was prepared from the resulting cells. Thereafter, the resulting nuclear extract, ³²P-labeled RNA probe of No. 5, and various amounts used of 2'-O-methyl oligo RNA (0.5 µg for lane 3, 5 µg for lane 4, 50 µg for lane 5, and 500 µg for lane 6) were incubated at 25°C for 30 minutes. The bound probe was separated from the free probe by electrophoresis at a constant voltage of 11 V/cm for 1.5 hours at 4°C on 4% polyacrylamide gel in a buffer (pH 8.5) containing 50 mM Tris, 0.38 M glycine and 2 mM EDTA. Thereafter, the gel after the electrophoresis was fixed and dried. Then, the dried gel was exposed to an X-ray film and subjected to autoradiography. The results are shown in Fig. 25.

As shown in lanes 1 and 2 in Fig. 25, the binding of HMG-I to the RNA probe of No. 5 having the sequence shown in SEQ ID NO: 3 was increased more in the nuclear extract of the SK-N-SH cells cultured under hypoxic conditions than in that of the SK-N-SH cells cultured under normoxia conditions. Since by adding the above 2'-O-methyl oligo RNA, the increase in this binding was inhibited in a dose-dependent manner, it was found that the above 2'-O-methyl oligo RNA inhibited the hypoxia-induced activity of binding between HMG-I and the target site on PS2 pre-mRNA.

### Example 12

By using OligofectAmine, ³²P-labeled 2'-O-methyl oligo RNA (SEQ ID NO: 27) was introduced into the SK-N-SH cells. Then, the whole lysate, the nuclear fraction and the cytosol fraction were extracted respectively. Thereafter, by measuring radioactivities for the whole lysate, the nuclear fraction and the cytosol fraction, respectively, the stability of 2'-O-methyl oligo RNA (SEQ ID NO: 27) in the SK-N-SH cells and the efficiency of introduction of 2'-O-methyl oligo RNA into the SK-N-SH cells were determined. The results are shown in panel A in Fig. 26.

As shown in a in panel A in Fig. 26, it was found that the 2'-O-methyl oligo RNA was introduced into the SK-N-SH cells without disappearance thereof and transferred to the nuclei in a dose-dependent manner. As shown in b in panel A in Fig. 26, it was found that the efficiency of introduction of the above 2'-O-methyl oligo RNA into the SK-N-SH cells was highest where the 2'-O-methyl oligo RNA was used in an amount of 20 µg/10 cm-dish.

However, it was estimated from panel A in Fig. 26 that the amount of the 2'-O-methyl oligo RNA incorporated into the cells was about 15 µg/10 cm-dish, so that the maximum dose for introduction into the cells was established to be 15 µg/10 cm-dish in the subsequent experiment.

48 hours after introduction of the 2'-O-methyl oligo RNA into the SK-N-SH cells, the stability of the 2'-O-methyl oligo RNA introduced into the nuclei was studied. The results are shown in panel B in Fig. 26.

As shown in panel B in Fig. 26, it was found that even 48 hours after introduction, the 2'-O-methyl oligo RNA had almost the same molecular weight as in the original fraction; that is, the 2'-O-methyl oligo RNA was stable in the nuclei without being decomposed even 48 hours after introduction.

### Example 13

The action of the 2'-O-methyl oligo RNA (SEQ ID NO: 27) on the binding of HMG-I to DNA in a nuclear extract derived from the SK-N-SH cells was studied.

First, the nucleotide sequence of the HMG-I recognition motif was determined by an *in vitro* DNA screening assay. The procedure of the *in vitro* DNA screening assay is as follows: A synthetic DNA containing a random sequence of 31 nucleotides (20.7% A, 22.7% C, 31.5% T and 25.1% G, as determined by direct sequencing of 16 clones)
[5'-GGTGATCAGATTCTGATCCA(N31)TGAAGCTTGGATCCGTCGC-3']
was amplified by using
5'-GTAATACGACTCACTATAGGGTGATCAGATTCTGATCCA-3'
(DRQ ID NO: 67) and 5'-GCGACGGATCCAAGCTTCA-3' (SEQ ID NO: 68) as primers in 7 cycles of PCR, wherein one cycle consists of reactions at 94°C for 1 minute, 53°C for 1 minute and 72°C for 1 minute.

The resulting product, together with recombinant HMG-I (referred to as rHMG-I) expressed in *E. coli*, was incubated at 25°C for 30 minutes in an incubation buffer [12 mM HEPES-NaOH (pH 7.9) containing 60 mM KCI, 4 mM MgCl₂, 1 mM EDTA, 1 mM EGTA, 1 mM DTT, 10% glycerol and 1 mM PMSF].

The resulting reaction solution was immunoprecipitated by using an antibody against HMG-I. Thereafter, the immunoprecipitated product was used as the template and
5'-GTAATACGACTCACTATAGGGTGATCAGATTCTGATCCA-3' (SEQ ID NO: 67) and 5'-GCGACGGATCCAAGCTTCA-3' (SEQ ID NO: 68) were used as primers to carry out 7 cycles of PCR, wherein one cycle consists of reactions at 94°C for 1 minute, 53°C for 1 minute and 72°C for 1 minute. The resulting PCR product was cloned into pGEM-T vector (manufactured by Promega) and analyzed by direct sequencing.

As a result, it was suggested that a consensus sequence shown in the uppermost position in Fig. 27 is the HMG-I recognition motif.

Then, the action of the 2'-O-methyl oligo RNA (SEQ ID NO: 27) on the binding of a ³²P-labeled DNA probe [gcgG(A)GT(A)A(T)ATTTcgc (SEQ ID NO: 66)] containing the HMG-I recognition motif to HMG-I was studied.

After the protein content in the nuclear extract was measured, an extract of an amount equivalent to 5 µg protein, 1 µg of poly dIdC, and the 2'-O-methyl oligo RNA (SEQ ID NO: 27) at each concentration were added to the above incubation buffer, and then 1 µg of the ³²P-labeled DNA probe [gcgG(A)GT(A)A(T)ATTTcgc (SEQ ID NO: 66)] was added thereto to make up to a total volume of 50 µl solution which was then incubated at 25°C for 30 minutes.

Thereafter, the bound probe was separated from the free probe by electrophoresis at a constant voltage of 11 V/cm for 1.5 hours at 4°C on 4% polyacrylamide gel in a buffer containing 50 mM Tris, 0.38 M glycine and 2 mM EDTA. The gel after electrophoresis was dried and analyzed by autoradiography.

As a result, as shown in the respective lane 1 and lane 2 in panels A and B in Fig. 28 respectively, the binding between the ³²P-labeled DNA probe containing the HMG-I recognition motif and HMG-I was significantly increased by overexpression of HMG-I in the SK-N-SH cells.

However, as shown in the respective lanes 3 and 4 in panels A and B in Fig. 28, no significant influence of the above 2'-O-methyl oligo RNA on the increase in the binding activity of HMG-I to the above DNA probe was observed.

Accordingly, it was suggested that the inhibitory action of the above 2'-O-methyl oligo RNA was specific to the binding of HMG-I to RNA, but not specific to the binding of HMG-I to DNA.

### Example 14

SK-N-SH cells [cells to be tested] with introduction of the 2'-O-methyl oligo RNA (SEQ ID NO: 27) and SK-N-SH cells without the introduction of the RNA [control cells] were cultured respectively at 37°C for 21 hours under hypoxic conditions or under normoxia conditions in an incubator equipped with a hypoxia chamber maintained in a humidified atmosphere at low oxygen pressure (8 torr), and then cultured for 10 hours in the presence or absence of 2 µM tunicamycin.

Thereafter, the survival rate of the cells was analyzed by 3-(4,5-dimethylthiazol-2-yl)-5-(3-carboxymethoxyphenyl)-2-(4-sulfophenyl)-2H-tetrazolium assay (referred to as MTS assay) using a commercial product from Promega, that is, Cell Titer 96^{R} AQueous One Solution Proliferation Assay.

As a result, as shown in lanes 4 to 7 in panel A in Fig. 29, it was found that the cell death induced by stimulation with low oxygen and tunicamycin was reduced in a dose-depending manner by introducing the above 2'-O-methyl oligo RNA.

In addition, SK-N-SH cells [cells to be tested] transiently expressing HMG-I into which the 2'-O-methyl oligo RNA (SEQ ID NO: 27) was introduced, the SK-N-SH cells transiently expressing HMG-I, and the SK-N-SH cells were cultured respectively for 10 hours in the presence or absence of 2 µM tunicamycin.

Thereafter, the survival rate of the cells was analyzed in the same manner as above by the MTS assay.

As a result, as shown in lanes 4 to 7 in panel B in Fig. 29, it was found that the cell death induced by transient expression of HMG-I and by tunicamycin was reduced significantly in a dose-depending manner by introducing the above 2'-O-methyl oligo RNA.

### SEQUENCE LISTING FREE TEXT

SEQ ID NO: 1 shows a sequence of a binding region.
SEQ ID NO: 2 shows a sequence of a binding region.
SEQ ID NO: 3 shows a sequence of RNA probe No. 5.
SEQ ID NO: 4 shows a sequence of RNA probe No. 7.
SEQ ID NO: 5 shows a sequence of RNA probe No. 9.
SEQ ID NO: 6 shows a sequence of RNA probe No. 10.
SEQ ID NO: 7 shows a sequence of RNA probe No. 11.
SEQ ID NO: 8 shows a sequence of RNA probe No. 8.
SEQ ID NO: 9 shows a sequence of primer ps251.
SEQ ID NO: 10 shows a sequence of primer ps231.
SEQ ID NO: 11 shows a sequence of primer ps252.
SEQ ID NO: 12 shows a sequence of primer ps233.
SEQ ID NO: 13 shows a sequence of DNA probe No. 1.
SEQ ID NO: 14 shows a sequence of DNA probe No. 2.
SEQ ID NO: 15 shows a sequence of DNA probe No. 3.
SEQ ID NO: 16 shows a sequence of DNA probe No. 4.
SEQ ID NO: 17 shows a sequence of DNA probe No. 5.
SEQ ID NO: 18 shows a sequence of DNA probe No. 6.
SEQ ID NO: 19 shows a sequence of DNA probe No. 7.
SEQ ID NO: 20 shows a sequence of DNA probe No. 8.
SEQ ID NO: 21 shows a sequence of DNA probe No. 9.
SEQ ID NO: 22 shows a sequence of DNA probe No. 10.
SEQ ID NO: 23 shows a sequence of DNA probe No. 11.
SEQ ID NO: 24 shows a sequence of 5'-amino-2'-O-methyl oligo RNA used as a ligand in an affinity column.
SEQ ID NO: 25 shows a sequence of RNA probe No. 6.
SEQ ID NO: 26 shows a sequence of RNA probe No. 12.
SEQ ID NO: 27 shows a sequence of the 2'-O-methyl oligo RNA corresponding to the sequence of the HMG-I protein binding region present in the SEQ ID NO: 24.
SEQ ID NO: 28 shows a sequence of a synthetic DNA containing the HMG-I recognition motif.
SEQ ID NO: 29 shows a sequence of a synthetic DNA containing the HMG-I recognition motif.
SEQ ID NO: 30 shows a sequence of a synthetic DNA containing the HMG-I recognition motif.
SEQ ID NO: 31 shows a sequence of a synthetic DNA containing the HMG-I recognition motif.
SEQ ID NO: 32 shows a sequence of a synthetic DNA containing the HMG-I recognition motif.
SEQ ID NO: 33 shows a sequence of a synthetic DNA containing the HMG-I recognition motif.
SEQ ID NO: 34 shows a sequence of a synthetic DNA containing the HMG-I recognition motif.
SEQ ID NO: 35 shows a sequence of a synthetic DNA containing the HMG-I recognition motif.
SEQ ID NO: 36 shows a sequence of a synthetic DNA containing the HMG-I recognition motif.
SEQ ID NO: 37 shows a sequence of a synthetic DNA containing the HMG-I recognition motif.
SEQ ID NO: 38 shows a sequence of a synthetic DNA containing the HMG-I recognition motif.
SEQ ID NO: 39 shows a sequence of a synthetic DNA containing the HMG-I recognition motif.
SEQ ID NO: 40 shows a sequence of a synthetic DNA containing the HMG-I recognition motif.
SEQ ID NO: 41 shows a sequence of a synthetic DNA containing the HMG-I recognition motif.
SEQ ID NO: 42 shows a sequence of a synthetic DNA containing the HMG-I recognition motif.
SEQ ID NO: 43 shows a sequence of a synthetic DNA containing the HMG-I recognition motif.
SEQ ID NO: 44 shows a sequence of a synthetic DNA containing the HMG-I recognition motif.
SEQ ID NO: 45 shows a sequence of a synthetic DNA containing the HMG-I recognition motif.
SEQ ID NO: 46 shows a sequence of a synthetic DNA containing the HMG-I recognition motif.
SEQ ID NO: 47 shows a sequence of a synthetic DNA containing the HMG-I recognition motif.
SEQ ID NO: 48 shows a sequence of a synthetic DNA containing the HMG-I recognition motif.
SEQ ID NO: 49 shows a sequence of a synthetic DNA containing the HMG-I recognition motif.
SEQ ID NO: 50 shows a sequence of a synthetic DNA containing the HMG-I recognition motif.
SEQ ID NO: 51 shows a sequence of a synthetic DNA containing the HMG-I recognition motif.
SEQ ID NO: 52 shows a sequence of a synthetic DNA containing the HMG-I recognition motif.
SEQ ID NO: 53 shows a sequence of a synthetic DNA containing the HMG-I recognition motif.
SEQ ID NO: 54 shows a sequence of a synthetic DNA containing the HMG-I recognition motif.
SEQ ID NO: 55 shows a sequence of a synthetic DNA containing the HMG-I recognition motif.
SEQ ID NO: 56 shows a sequence of a synthetic DNA containing the HMG-I recognition motif.
SEQ ID NO: 57 shows a sequence of a synthetic DNA containing the HMG-I recognition motif.
SEQ ID NO: 58 shows a sequence of a synthetic DNA containing the HMG-I recognition motif.
SEQ ID NO: 59 shows a sequence of a synthetic DNA containing the HMG-I recognition motif.
SEQ ID NO: 60 shows a sequence of a synthetic DNA containing the HMG-I recognition motif.
SEQ ID NO: 61 shows a sequence of a synthetic DNA containing the HMG-I recognition motif.
SEQ ID NO: 62 shows a sequence of a synthetic DNA containing the HMG-I recognition motif.
SEQ ID NO: 63 shows a sequence of a synthetic DNA containing the HMG-I recognition motif.
SEQ ID NO: 64 shows a sequence of a synthetic DNA containing the HMG-I recognition motif.
SEQ ID NO: 65 shows a sequence of a synthetic DNA used for *in vitro* DNA screening assay. n represents G, A, T or C.
SEQ ID NO: 66 shows a sequence of a synthetic DNA containing the HMG-I recognition motif.
SEQ ID NO: 67 shows a sequence of a primer used for *in vitro* DNA .screening assay.
SEQ ID NO: 68 shows a sequence of a primer used for *in vitro* DNA screening assay.

### INDUSTRIAL APPLICABILITY

According to the inhibitor of the present invention, there is exhibited an excellent effect that the inhibitor can inhibit a binding between the HMG-I protein and exon 5 of presenilin-2 mRNA, whereby a disease caused by generation of an aberrant splicing that lacks exon 5 of presenilin-2 mRNA, particularly a brain disorder or neurodegenerative disease can be treated or prevented. Also, according to the agent for suppressing neuronal death, there is exhibited an excellent effect that an aberrant splicing that lacks exon 5 of presenilin-2 can be suppressed, thereby suppressing neuronal death, whereby treatment or prevention of a disease caused by generation of a splicing variant that lacks exon 5 of presenilin-2 mRNA, particularly a brain disorder or neurodegenerative disease can be carried out. Further, according to the pharmaceutical composition of the present invention, there is exhibited an excellent effect that neuronal death can be suppressed, whereby treatment or prevention of a disease caused by generation of a splicing variant that lacks exon 5 of presenilin-2 mRNA, particularly a brain disorder or neurodegenerative disease can be carried out. In addition, according to the method of treatment or prevention of the present invention, there is exhibited an excellent effect that treatment or prevention of a disease caused by generation of a splicing variant that lacks exon 5 of presenilin-2 mRNA, particularly a brain disorder or neurodegenerative disease can be carried out even more efficiently and even more sustainedly.

## Claims

1. An inhibitor for a binding between HMG-I protein and presenilin-2 gene, comprising a compound capable of inhibiting a binding between HMG-I protein and exon 5 of presenilin-2 mRNA as an active ingredient.

2. The inhibitor according to claim 1, wherein the compound is one kind selected from the group consisting of:
(A) an RNA molecule comprising the nucleotide sequence shown in SEQ ID NO: 1 and/or the nucleotide sequence shown in SEQ ID NO: 2, wherein the RNA molecule is capable of binding to the HMG-I protein;
(B) a DNA molecule comprising a DNA sequence corresponding to the nucleotide sequence shown in SEQ ID NO: 1 and/or a DNA sequence corresponding to the nucleotide sequence shown in SEQ ID NO: 2, wherein the DNA molecule is capable of binding to the HMG-I protein;
(C) an antisense molecule complementary to either the RNA molecule of the above (A) or the DNA molecule of the above (B);
(D) an analog molecule which is an analog of either the RNA molecule of the above (A) or the DNA molecule of the above (B), wherein the analog molecule is capable of binding to the HMG-I protein; and
(E) an analog molecule of the antisense molecule of the above (C).

3. The inhibitor according to claim 2, wherein the compound is one kind selected from the group consisting of:
(a) an RNA molecule comprising the nucleotide sequence shown in any one of SEQ ID NO: 3, 4, 5, 6 or 7, wherein the RNA molecule is capable of binding to the HMG-I protein;
(b) a DNA molecule comprising a DNA sequence corresponding to the nucleotide sequence shown in any one of SEQ ID NO: 3, 4, 5, 6 or 7, wherein the DNA molecule is capable of binding to the HMG-I protein;
(c) an antisense molecule complementary to either the RNA molecule of the above (a) or the DNA molecule of the above (b);
(d) an analog molecule which is an analog of either the RNA molecule of the above (a) or the DNA molecule of the above (b), wherein the analog molecule is capable of binding to the HMG-I protein; and
(e) an analog molecule of the antisense molecule of the above (c).

4. The inhibitor according to claim 1 or 2, wherein the compound is 2'-O-methyl oligo RNA molecule having the nucleotide sequence shown in SEQ ID NO: 27.

5. An agent for suppressing neuronal death, comprising as an active ingredient: an inhibitor for a binding between HMG-I protein and presenilin-2 gene, wherein the inhibitor comprises a compound capable of inhibiting a binding between HMG-I protein and exon 5 of presenilin-2 mRNA as an active ingredient.

6. The agent for suppressing neuronal death according to claim 5, wherein the compound is one kind selected from the group consisting of:
(A) an RNA molecule comprising the nucleotide sequence shown in SEQ ID NO: 1 and/or the nucleotide sequence shown in SEQ ID NO: 2, wherein the RNA molecule is capable of binding to the HMG-I protein;
(B) a DNA molecule comprising a DNA sequence corresponding to the nucleotide sequence shown in SEQ ID NO: 1 and/or a DNA sequence corresponding to the nucleotide sequence shown in SEQ ID NO: 2, wherein the DNA molecule is capable of binding to the HMG-I protein;
(C) an antisense molecule complementary to either the RNA molecule of the above (A) or the DNA molecule of the above (B);
(D) an analog molecule which is an analog of either the RNA molecule of the above (A) or the DNA molecule of the above (B), wherein the analog molecule is capable of binding to the HMG-I protein; and
(E) an analog molecule of the antisense molecule of the above (C).

7. The agent for suppressing neuronal death according to claim 6, wherein the compound is one kind selected from the group consisting of:
(a) an RNA molecule comprising the nucleotide sequence shown in any one of SEQ ID NO: 3, 4, 5, 6 or 7, wherein the RNA molecule is capable of binding to the HMG-I protein;
(b) a DNA molecule comprising a DNA sequence corresponding to the nucleotide sequence shown in any one of SEQ ID NO: 3, 4, 5, 6 or 7, wherein the DNA molecule is capable of binding to the HMG-I protein;
(c) an antisense molecule complementary to either the RNA molecule of the above (a) or the DNA molecule of the above (b);
(d) an analog molecule which is an analog of either the RNA molecule of the above (a) or the DNA molecule of the above (b), wherein the analog molecule is capable of binding to the HMG-I protein; and
(e) an analog molecule of the antisense molecule of the above (c).

8. The agent for suppressing neuronal death according to claim 5 or 6, wherein the compound is 2'-O-methyl oligo RNA molecule having the nucleotide sequence shown in SEQ ID NO: 27.

9. A pharmaceutical composition comprising as an active ingredient:
an inhibitor for a binding between HMG-I protein and presenilin-2 gene, comprising a compound capable of inhibiting a binding between HMG-I protein and exon 5 of presenilin-2 mRNA as an active ingredient; or
an agent for suppressing neuronal death, comprising the inhibitor as an active ingredient.

10. The pharmaceutical composition according to claim 9, wherein the compound is one kind selected from the group consisting of:
(A) an RNA molecule comprising the nucleotide sequence shown in SEQ ID NO: 1 and/or the nucleotide sequence shown in SEQ ID NO: 2, wherein the RNA molecule is capable of binding to the HMG-I protein;
(B) a DNA molecule comprising a DNA sequence corresponding to the nucleotide sequence shown in SEQ ID NO: 1 and/or a DNA sequence corresponding to the nucleotide sequence shown in SEQ ID NO: 2, wherein the DNA molecule is capable of binding to the HMG-I protein;
(C) an antisense molecule complementary to either the RNA molecule of the above (A) or the DNA molecule of the above (B);
(D) an analog molecule which is an analog of either the RNA molecule of the above (A) or the DNA molecule of the above (B), wherein the analog molecule is capable of binding to the HMG-I protein; and
(E) an analog molecule of the antisense molecule of the above (C).

11. The pharmaceutical composition according to claim 10, wherein the compound is one kind selected from the group consisting of:
(a) an RNA molecule comprising the nucleotide sequence shown in any one of SEQ ID NO: 3, 4, 5, 6 or 7, wherein the RNA molecule is capable of binding to the HMG-I protein;
(b) a DNA molecule comprising a DNA sequence corresponding to the nucleotide sequence shown in any one of SEQ ID NO: 3, 4, 5, 6 or 7, wherein the DNA molecule is capable of binding to the HMG-I protein;
(c) an antisense molecule complementary to either the RNA molecule of the above (a) or the DNA molecule of the above (b);
(d) an analog molecule which is an analog of either the RNA molecule of the above (a) or the DNA molecule of the above (b), wherein the analog molecule is capable of binding to the HMG-I protein; and
(e) an analog molecule of the antisense molecule of the above (c).

12. The pharmaceutical composition according to claim 9 or 10, wherein the compound is 2'-O-methyl oligo RNA molecule having the nucleotide sequence shown in SEQ ID NO: 27.

13. The pharmaceutical composition according to any one of claims 9 to 12, wherein the pharmaceutical composition possesses an action of suppressing aberrant splicing.

14. The pharmaceutical composition according to any one of claims 9 to 13, wherein the pharmaceutical composition possesses an action of suppressing generation of a splicing variant that lacks exon 5 of presenilin-2 mRNA.

15. The pharmaceutical composition according to any one of claims 9 to 14, wherein the pharmaceutical composition is usable for treating or preventing a brain disorder or a neurodegenerative disease caused by generation of a splicing variant that lacks exon 5 of presenilin-2 mRNA.

16. The pharmaceutical composition according to claim 15, wherein the brain disorder is an ischemic brain disorder or a brain neurodegenerative disease.

17. The pharmaceutical composition according to claim 16, wherein the brain disorder is a disease selected from the group consisting of cerebrovascular dementia, Parkinson's syndrome and Alzheimer disease.

18. The pharmaceutical composition according to claim 15, wherein the neurodegenerative disease is amyotrophic lateral sclerosis.

19. Use of the inhibitor of any one of claims 1 to 4 or the agent for suppressing neuronal death of any one of claims 5 to 8, for manufacture of a pharmaceutical composition for administering to an individual with a disease caused by generation of a splicing variant that lacks exon 5 of presenilin-2 mRNA, thereby treating or preventing the disease caused by generation of a splicing variant that lacks exon 5 of presenilin-2 mRNA.

20. A method for treating or preventing a disease caused by generation of a splicing variant that lacks exon 5 of presenilin-2 mRNA, **characterized by** administering the inhibitor of any one of claims 1 to 4 or the agent for suppressing neuronal death of any one of claims 5 to 8 in a therapeutically effective amount to an individual with a disease caused by generation of a splicing variant that lacks exon 5 of presenilin-2 mRNA.
